# EUROPEAN PATENT SPECIFICATION

(11) **EP 4 061 368 B1**
(45) Date of publication and mention of the grant of the patent: **15.07.2026**
(21) Application number: 20824753.6
(22) Date of filing: 17.11.2020
(51) Int. Cl.: A61K 31/4439, A61P 7/00

(54) **METHODS OF ADMINISTERING VOXELOTOR**
VERFAHREN ZUR VERABREICHUNG VON VOXELOTOR
PROCÉDÉS D'ADMINISTRATION DE VOXELOTOR

(30) Priority: 19.11.2019 US 201962937706 P; 25.11.2019 US 201962940154 P
(43) Date of publication of application: 28.09.2022
(73) Proprietor: Global Blood Therapeutics, Inc., South San Francisco, CA 94080 (US)
(72) Inventor: WASHINGTON, Carla B., San Francisco, California 94080 (US)
(74) Representative: Pfizer
(86) International application number: PCT/US2020/060923
(87) International publication number: WO 2021/101910

(56) References cited:
- "Single dose PK study of GBT440 in Subjects with renal Impairment.", 19 May 2019 (2019-05-19), XP002802162, Retrieved from the Internet <URL:https://clinicaltrials.gov/ct2/show/NCT03161015> [retrieved on 20210222]
- HOWARD JO ET AL: "A phase 1/2 ascending dose study and open-label extension study of voxelotor in patients with sickle cell disease", BLOOD, AMERICAN SOCIETY OF HEMATOLOGY NLD, US, vol. 133, no. 17, 25 April 2019 (2019-04-25), pages 1865 - 1875, XP009525831, ISSN: 1528-0020, DOI: 10.1182/BLOOD-2018-08-868893
- HUTCHALEELAHA ATHIWAT ET AL: "Pharmacokinetics and pharmacodynamics of voxelotor (GBT440) in healthy adults and patients with sickle cell disease", BRITISH JOURNAL OF CLINICAL PHARMACOLOGY, BLACKWELL PUBLISHING, GB, vol. 85, no. 6, 31 May 2019 (2019-05-31), pages 1290 - 1302, XP009525830, ISSN: 1365-2125, DOI: 10.1111/BCP.13896
- WONG W Y ET AL: "Renal failure in sickle cell anemia", vol. 10, no. 6, 30 November 1996 (1996-11-30), pages 1321 - 1331, XP009525832, ISSN: 0889-8588, Retrieved from the Internet <URL:https://api.elsevier.com/content/article/PII:S0889858805704032?httpAccept=text/plain> DOI: 10.1016/S0889-8588(05)70403-2

## Description

### FIELD

Provided herein is voxelotor for use in methods for treating sickle cell disease in patients that also have severe hepatic impairment.

### BACKGROUND

Voxelotor (also known as Oxbryta^{™} and formerly known as GBT440) is a small molecule allosteric modifier of hemoglobin-oxygen affinity useful for the treatment of sickle cell disease (SCD). Voxelotor can be administered orally. Voxelotor can increase hemoglobin's affinity for oxygen, thereby stabilizing hemoglobin in the oxyhemoglobin state, which can lead to inhibition of polymerization of sickle hemoglobin (HbS).

The major route of elimination of voxelotor is by metabolism. In sickle cell patients with normal hepatic function, 1500 mg of voxelotor significantly increased hemoglobin levels and reduced markers of hemolysis, indicating inhibition of HbS polymerization. In patient populations with severe, moderate, or mild hepatic impairment, drug dosages may or may not need to be adjusted for such patients. Thus, there is a need for developing treatment suitable for SCD patients who also have hepatic impairment.

Further, voxelotor is also a moderate inhibitor of cytochrome P450 (CYP)3A4, the most prevalent CYP enzyme in the liver, in humans. Accordingly, improved methods for administering voxelotor are needed to avoid or lessen potential adverse drug interactions.

Howard et al. (Blood, 2019, 133(17), pages 1865-1875) describes a phase 1/2 ascending dose study and open-label extension study of voxelotor in patients with sickle cell disease. All patients who received multiple doses of voxelotor for ≥ 28 days experienced hematological improvements including increased haemaglobin and reduction in hemolysis and percentage of sickled red cells (abstract). Exclusion criteria applied (page 1866).

### SUMMARY

Provided herein is voxelotor for use in a method of treating sickle cell disease in a patient in need thereof. The method comprises administering to a patient having sickle cell disease 500 mg to 1000 mg of voxelotor per day, wherein the patient also has severe hepatic impairment. Some embodiments provide voxelotor for use in a method of treating sickle cell disease in a patient in need thereof, the method comprising administering to the patient 1000 mg of voxelotor per day, wherein the patient has severe hepatic impairment.

### BRIEF DESCRIPTION OF THE DRAWINGS

FIG. 1 shows plots for mean (+SD) of whole blood voxelotor concentration-time profiles (pharmacokinetic evaluable population).
FIG. 2A, FIG. 2B, FIG. 2C, and FIG. 2D show plots for overlay of whole blood voxelotor concentration-time profile (pharmacokinetic full population) for hepatic impairment status of subjects being mild (FIG. 2A), moderate (FIG. 2B), severe (FIG. 2C), and severe with dose adjusted (FIG. 2D).
FIG. 3 shows plots for mean (+ SD) of plasma voxelotor concentration-time profiles (pharmacokinetic evaluation population).
FIG. 4A, FIG. 4B, FIG. 4C, and FIG. 4D show plots for overlay of plasma voxelotor concentration-time profile (pharmacokinetic full population) for hepatic impairment status of subjects being mild (FIG. 4A), moderate (FIG. 4B), severe (FIG. 4C), and severe with dose adjusted (FIG. 4D).
FIG. 5 shows forest plots of individual ratios of pharmacokinetics parameters of whole blood voxelotor by hepatic impairment status group (pharmacokinetic evaluable population).
FIG. 6 shows forest plots of individual ratios of pharmacokinetics parameters of plasma voxelotor by hepatic impairment status group (pharmacokinetic evaluable population).
FIG. 7 shows a forest plot to assess the effect of multiple doses of voxelotor on the pharmacokinetics of caffeine, S-warfarin, omeprazole, midazolam, and their metabolites.
FIG. 8 shows subject-level change from baseline in hemoglobin at week 24 in patients who completed 24 weeks of treatment. Approximately 82% of all randomized patients completed 24 weeks of treatment.

### DETAILED DESCRIPTION

### Definitions

Unless defined otherwise, all technical and scientific terms used herein have the same meaning as is commonly understood by one of ordinary skill in the art. As used herein, the below terms have the following meanings unless specified otherwise. Any methods, devices and materials similar or equivalent to those described herein can be used in the practice of the compositions and methods described herein.

It is noted here that as used in this specification and the appended claims, the singular forms "a" "an" and "the" and the like include plural referents unless the context clearly dictates otherwise.

The term "about" or "approximately" means within ± 30%, 20%, 15%, 10%, 9%, 8%, 7%, 6%, 5%, 4%, 3%, 2%, 1%, 0.5%, or 0.05% of a given value or range. In some embodiments, "about" means ± 5% of a given value or range. In some embodiments, "about" means ± 4% of a given value or range. In some embodiments, "about" means ± 3% of a given value or range. In some embodiments, "about" means ± 2% of a given value or range. In some embodiments, "about" means ± 1% of a given value or range. In another embodiment, about means ± 0.5% of a given value or range. In some embodiments, "about" means ± 0.05% of a given value or range.

As used herein, the term "administration" refers to introducing an agent into a patient. For example, a therapeutic amount can be administered to the patient, which can be determined by the treating physician or the like. In some embodiments, an oral route of administration is preferred. The related terms and phrases "administering" and "administration of," when used in connection with a compound or tablet (and grammatical equivalents) refer both to direct administration, which may be administration to a patient by a medical professional or by self-administration by the patient, and/or to indirect administration, which may be the act of prescribing a drug. Administration entails delivery to the patient of the drug.

The term "dose" or "dosage" refers to the total amount of an active agent (e.g., voxelotor) administered to a patient in a single day (24-hour period). The desired dose can be administered once daily. In some embodiments, the desired dose may be administered in one, two, three, four or more sub-doses at appropriate intervals throughout the day, where the cumulative amount of the sub-doses equals the amount of the desired dose administered in a single day. The terms "dose" and "dosage" are used interchangeably herein.

As used herein, where the mass of voxelotor is specified, for example, 500 mg, 1000 mg or 1500 mg of voxelotor, that amount corresponds to the mass of voxelotor in its free base form in a single tablet.

The term "hemoglobin" as used herein refers to any hemoglobin protein, including normal hemoglobin (Hb) and sickle hemoglobin (HbS).

The term "sickle cell disease" refers to diseases mediated by sickle hemoglobin (HbS) that results from a single point mutation in the hemoglobin (Hb). Non-limiting examples of sickle cell diseases include sickle cell anemia, sickle-hemoglobin C disease (HbSC), sickle beta-plus-thalassaemia (HbS/β) and sickle beta-zero-thalassaemia (HbS/β0).

As used herein, "therapeutically effective amount" or "therapeutic amount" refers to an amount of a drug or an agent (e.g., voxelotor) that when administered to a patient suffering from a condition, will have the intended therapeutic effect, e.g., alleviation, amelioration, palliation or elimination of one or more manifestations of the condition in the patient. The full therapeutic effect does not necessarily occur by administration of one dose, and can occur only after administration of a series of doses and can be administered in one dose form or multiples thereof. For example, 1000 mg of the drug can be administered in a single 1000 mg strength tablet or two 500 mg strength tablets. As another example, 500 mg of the drug can be administered in a single 500 mg strength tablet. Thus, a therapeutically effective amount may be administered in one or more administrations. For example, and without limitation, a therapeutically effective amount of an agent, in the context of treating disorders related to hemoglobin S, refers to an amount of the agent that alleviates, ameliorates, palliates, or eliminates one or more manifestations of the disorders related to hemoglobin S in the patient.

As used herein, the term "pharmaceutically acceptable" refers to generally safe and non-toxic for *in vivo,* preferably human, administration.

As used herein, the term "patient" refers to a mammal, such as a human, bovine, rat, mouse, dog, monkey, ape, goat, sheep, cow, or deer. A patient as described herein can be a human. In some embodiments, the patient is an adult. In some embodiments, the patient is a child or juvenile. In some embodiments, the patient is about 9 months old to about 11 years old.

As used herein, "treatment," "treating," and "treat" are defined as acting upon a disease, disorder, or condition with an agent to reduce or ameliorate the harmful or any other undesired effects of the disease, disorder, or condition and/or its symptoms. Treatment, as used herein, covers the treatment of a human patient, and includes: (a) reducing the risk of occurrence of the condition in a patient determined to be predisposed to the disease but not yet diagnosed as having the condition, (b) impeding the development of the condition, and/or (c) relieving the condition, i.e., causing regression of the condition and/or relieving one or more symptoms of the condition. For purposes of treatment of sickle cell disease, beneficial or desired clinical results include, but are not limited to, multi-lineage hematologic improvement, decrease in the number of required blood transfusions, decrease in infections, decreased bleeding, and the like. For purposes of treatment of interstitial pulmonary fibrosis, beneficial or desired clinical results include, but are not limited to, reduction in hypoxia, reduction in fibrosis, and the like.

As used herein, "% w/w" refers to the weight of a component based on the total weight of a composition comprising the component. For instance, if component 1 is present in an amount of 50% in a 100 mg composition, component 1 is present in an amount of 50 mg. In some embodiments, the composition refers to a tablet as described herein.

### Voxelotor

Voxelotor is a small molecule allosteric modifier of hemoglobin-oxygen affinity in clinical development stage for the treatment of sickle cell disease (SCD). Voxelotor increases hemoglobin's affinity for oxygen, thereby stabilizing hemoglobin in the oxyhemoglobin state, which leads to inhibition of polymerization of sickle hemoglobin. The chemical name for voxelotor is 2-hydroxy-6-((2-(1-isopropyl-1H-pyrazol-5-yl)pyridin-3-yl)methoxy)benzaldehyde. Voxelotor has the structure as shown in the following:

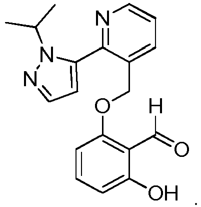

The synthesis of voxelotor is described in U.S. Patent No. 9,018,210 and U.S. Patent No. 10,077,249.

The crystalline solid form of voxelotor includes, for example, crystalline Form I, Form II or Form N as disclosed in PCT Application Publication No. WO 2015/120133.

In some embodiments, voxelotor is an ansolvate crystalline form characterized by at least two, three or four X-ray powder diffraction peaks (Cu Kα radiation) selected from 13.37°, 14.37°, 19.95° and 23.92°2θ (each ±0.2 °2θ). In some embodiments, voxelotor is an ansolvate crystalline form characterized by X-ray powder diffraction peaks (Cu Kα radiation) at 13.37°, 14.37°, 19.95° and 23.92°2θ (each ±0.2 °2θ). This form can hereinafter also be referred to as Form II.

In some embodiments, voxelotor is an ansolvate crystalline form characterized by at least two, three or four X-ray powder diffraction peaks (Cu Kα radiation) selected from 11.65°, 11.85°, 12.08°, 16.70°, 19.65° and 23.48 °2θ (each ±0.2 °2θ). In some embodiments, voxelotor is an ansolvate crystalline form characterized by X-ray powder diffraction peaks (Cu Kα radiation) at 11.65°, 11.85°, 12.08°, 16.70°, 19.65° and 23.48 °2θ (each ±0.2 °2θ). This form can hereinafter also be referred to as Form N or Material N.

In some embodiments, voxelotor is an ansolvate crystalline form characterized by at least two, three or four X-ray powder diffraction peaks (Cu Kα radiation) selected from 12.82°,15.74°, 16.03°, 16.63°, 17.60°, 25.14°, 25.82, and 26.44° °2θ (each ±0.2 °2θ). In some embodiments, voxelotor is an ansolvate crystalline form characterized by X-ray powder diffraction peaks (Cu Kα radiation) at 12.82°,15.74°, 16.03°, 16.63°, 17.60°, 25.14°, 25.82, and 26.44 °2θ (each ±0.2 °2θ). This form can hereinafter also be referred to as Form I.

### Hepatic Impairment

As used herein, a subject with "hepatic impairment" is a subject with a reduced hepatic function, for example a subject diagnosed with a clinical decrease in liver function. The reduced liver function can be caused by a liver disease suffered by the subject, for example hepatic encephalopathy, hepatitis, or cirrhosis. Sometimes, hepatic impairment can lead to liver failure. A number of methods have been developed to quantify hepatic functions and to determine the extent of hepatic impairment in patients, including a model end stage liver disease (MELD) score and a Child-Pugh score.

The Child-Pugh score (also known as the Child-Turcotte-Pugh score) is the most commonly used method to assess the prognosis of chronic liver disease, for example cirrhosis. It is an aggregate score of five clinical measures of liver disease: total bilirubin, serum albumin, prothrombin time prolongation or international normalized ratio (INR), ascites, and hepatic encephalopathy. Each marker is assigned a value from 1-3 points, with 3 indicating the most severe derangement. The total value of points is used to provide a score categorized as A (5-6 points), B (7-9 points), or C (10-15 points), which can be correlated with one and two year survival rates. Category A (i.e. Child-Pugh A score of 5-6 points) is considered as mild hepatic impairment, category B is considered to be moderate hepatic impairment (i.e. Child-Pugh B score of 7-9 points), and category C is considered to be severe hepatic impairment (i.e. Child-Pugh B score of 10-15 points). Methods for determination and analysis of Child-Pugh scores are well known in the art. Another commonly used scoring system to assess hepatic impairment, the Model for End-stage Liver Disease (MELD) was initially created to predict survival among patients undergoing transjugular intrahepatic portosystemic shunt (TIPS) placement. The MELD contains three objective variables: international normalized ratio (INR), creatinine, and total bilirubin. More recent but not uniformly adopted modification includes addition of sodium values.

As disclosed herein, any methods recognized in the art suitable to determine the severity of hepatic impairment of a subject can be used. In some embodiments, the hepatic impairment of the patient is determined by the Child-Pugh score. The patient, for example, can have a Child-Pugh score of, or about, 7, 8, 9, 10, 11, 12, 13, 14, 15, or a range between any two of these values, points. In some embodiments, the patient has a Child-Pugh score of, or about, 10, 11, 12, 13, 14, 15, or a range between any two of these values, points. In some embodiments, the patient has a Child-Pugh score of 10-15 points (i.e. category C).

Hepatic impairments in the patients can be caused by various conditions and reasons including, but not limited to liver diseases, cancers, autoimmune diseases, toxins, metabolic diseases, shock (e.g., sepsis, hepatic shock), vascular diseases (e.g., Budd-Chiari syndrome), hypoxemia, medication uses or overuses, and any combinations thereof. For example, the hepatic impairment of the patient can be caused by a liver disease, for example a chronic liver disease or an acute liver disease. The liver diseases can be caused by, for example, infections (e.g., parasite and/or viral infections), immune system abnormality, genetic abnormality, cancer and other growths, chronic alcohol abuse, and/or accumulation of fat in the liver. Examples of liver diseases include, but are not limited to, hepatitis (e.g., hepatitis A, hepatitis B, hepatitis C, hepatitis D, and hepatitis E), autoimmune hepatitis, alcoholic hepatitis, primary biliary cirrhosis, primary sclerosing cholangitis, hemochromatosis, hyperoxaluria and oxalosis, Wilson's disease, alpha-1 antitrypsin deficiency, liver cancer, bile duct cancer, liver adenoma, fatty liver disease (including alcoholic fatty liver disease and nonalcoholic fatty liver disease), cirrhosis, hypo-perfusion, or any combination thereof. In some embodiments, the hepatic impairment of the patient is caused by a physical injury to the liver. In some embodiments, the hepatic impairment of the patient is caused by liver inflammation. In some embodiments, the hepatic impairment of the patient is caused by the use or excess use of one or more drugs, for example acetaminophen, narcotic-acetaminophen combination medications, statin type of drugs for controlling elevated blood levels of cholesterol, niacin, antibiotics (e.g., nitrofurantoin, amoxicillin and clavulanic acid, tetracycline, and isoniazid), drugs for treating autoimmune disorders and/or cancers (e.g., methotrexate), drugs for treating alcoholics (e.g., disulfiram), or any combination thereof. In some embodiments, the hepatic impairment of the patient is caused by a use or an excess use of one or more herbal supplements, including but not limited to, kava, ephedra, skullcap and pennyroyal. In some embodiments, the hepatic impairment of the patient is caused by an excess use of vitamins (e.g., vitamin A). In some embodiments, the hepatic impairment of the patient is caused by food (e.g., mushrooms).

The chronic liver disease can be, for example, liver fibrosis, cirrhosis, end-stage liver disease (ESLD), hepatocellular carcinoma, hepatic steatosis (i.e., fat in the liver), nonalcoholic fatty liver disease (NAFLD), nonalcoholic steatohepatitis (NASH), portal hypertension, or any combination thereof. In some embodiments, the hepatic impairment is caused by chronic liver injury. In some embodiments, the hepatic impairment is caused by hepatic inflammation. Some patients can have ascites, bleeding diathesis, variceal bleeding, hepatic inflammation, renal dysfunction, hepatic encephalopathy, increased susceptibility to infection, and multi-organ dysfunction. The cirrhosis can be compensated or decompensated. In some embodiments, the patient having severe hepatic impairment suffers from a chronic hepatic disease. In some embodiments, the patient having severe hepatic impairment suffers from a chronic hepatic injury.

In some embodiments, the method further comprises identifying a patient having sickle cell disease that also has severe hepatic impairment. For example, clinical data or test results from a patient can be used to determine if a patient has sickle cell disease and severe hepatic impairment. In some embodiments, the identification can be made by a medical professional by using information obtained from the patient, information obtained from the patient's medical records, or information collected from test results. A medical professional having this information available to them and being able to identify subjects having sickle cell disease and severe hepatic impairment can practice the methods disclosed herein.

### Methods of Treatment for Patients with Hepatic Impairment

The present invention provides voxelotor for use in a method of treating sickle cell disease in a patient in need thereof wherein the patient also has severe hepatic impairment.

Whether dose adjustment is needed in patients with hepatic impairment can be difficult to predict. Further, the amount of dose adjustment needed is also difficult to predict, including for mild, moderate, or severe hepatic impairment patients.

It is contemplated that the dose of voxelotor for patients with normal liver function may need to be adjusted for patients with hepatic impairment.

A condition that can cause a severe hepatic impairment can be cancer, liver injury, autoimmune disease, drug overuse, or any combination thereof. The patient's age can vary. For example, the patient can be an adult, a child or juvenile. In some embodiments, the patient is at least 18 years old. In some embodiments, the patient is about 9 months old to about 11 years old.

The administration of voxelotor can be oral administration, for example in the form of capsules or tablets. The administration can be, for example, once daily, twice or three times a day, or once two days. In some embodiments, the administration is once daily. In some embodiments, the patient is administered one or two tablets described herein comprising a therapeutically effective amount of voxelotor per day.

In some embodiments, the dose of voxelotor is reduced to avoid a treatment-emergent adverse event. In some embodiments, the treatment-emergent adverse event is diarrhea. In some embodiments, the treatment-emergent adverse event is a headache. In some embodiments, the treatment-emergent adverse event is nausea, arthralgia, an upper respiratory tract infection, abdominal pain, fatigue, rash, pyrexia, pain in extremity, back pain, vomiting, pain, noncardiac chest pain, or upper abdominal pain. In some embodiments, the treatment-emergent adverse event is abdominal pain, diarrhea, nausea, fatigue, or pain. In some embodiments, the treatment-emergent adverse event is diarrhea, abdominal pain, nausea, fatigue, rash, or drug hypersensitivity.

In some embodiments, the method comprises administering to the patient two tablets described herein once daily, wherein each of the two tablets comprises about 500 mg of voxelotor.

In some embodiments, the methods comprise administering to the patient 500 mg of voxelotor per day. In some embodiments, the methods comprise administering to the patient 1000 mg of voxelotor per day. In some embodiments, the methods comprise administering to the patient one tablet of voxelotor per day, wherein the tablet comprises 500 mg voxelotor. In some embodiments, the methods comprise administering to the patient two tablets of voxelotor per day, wherein each of the two tablets comprises 500 mg voxelotor. In some embodiments, the methods comprise administering to the patient two tablets of voxelotor at once per day, wherein each of the two tablets comprises 500 mg voxelotor. In some embodiments, the methods comprise administering to the patient in a single oral dose of two tablets of voxelotor daily, wherein each of the two tablets comprises 500 mg voxelotor.

Some embodiments provide for voxelotor for use in methods of treating sickle cell disease in a patient in need thereof, the methods comprising administering to the patient 1000 mg of voxelotor once daily, wherein the patient has severe hepatic impairment.

Duration of the treatment can vary. For example, voxelotor can be administered to the patient for at least about, or for about, 20, 21, 22, 23, 24, 25, 26, 27, 28, 29, 30, 31, 32, 33, 34, 35, 36, or a range between any two of these values, days.

In some embodiments, voxelotor (e.g., the tablet(s) of voxelotor) is administrated to the patient with food. In some embodiments, voxelotor (e.g., the tablet of voxelotor) is administrated to the patient without food.

### Methods of Treatment to Avoid Drug-Drug Interactions ("DDI")

The cytochrome P450 (CYP) is a superfamily of enzymes responsible for the metabolic transformation of drugs, with CYP3A4 being the most prevalent CYP enzyme in the liver. CYP3A4 activity can be induced (or accelerated) or inhibited (decreased), which can impact drug concentrations. The inhibition of CYP3A4 can result in the accumulation of parent drug concentrations that can put the patient at increased risk for side effects and possible toxicity.

It has been found that voxelotor is a moderate inhibitor of CYP3A4 in humans, which may result in increased exposures of CYP3A4 substrates.

A "strong inhibitor" of a CYP enzyme as used herein is an inhibitor that caused a greater than 5-fold increase in the plasma AUC values or more than 80% decrease in clearance of CYP substrates (not limited to sensitive CYP substrate) in clinical evaluations.

A "moderate inhibitor" is an inhibitor that caused a less than 2-fold but greater than 5-fold increase in the AUC values or 50-80% decrease in clearance of sensitive CYP substrates when the inhibitor was given at the highest approved dose and the shortest dosing interval in clinical evaluations.

As used herein, a "sensitive CYP3A4 substrate" or "sensitive CYP3A4 substrate with a narrow therapeutic index" refers to a modulator of CYP3A4 where small differences in dose or blood concentration of the modulator may lead to dose and blood concentration dependent, serious therapeutic failures or adverse drug reactions. Non-limiting examples of a sensitive CYP3A4 substrate with a narrow therapeutic index include, but are not limited to, aminophylline, cyclobenzaprine, meperidine, temosirolimus, buspirone, rilpivirine, tadalafil, and dasatinib. In some embodiments, the sensitive CYP3A4 substrate with a narrow therapeutic index is alfentanil, cyclosporine, fentanyl, quinidine, sirolimus, or tacrolimus.

Non-limiting examples of a strong CYP3A4 inhibitor include, but are not limited to, VIEKIRA PAK (ombitasavir, partiaprevir, ritonavir and dasabuvir), indinavir/ritonavir, tipranavir, ritonavir, cobicistat, ketoconazole, indinavir, troleandomycin, telaprevir, danoprevir, elvitegravir/ritonavir, saquinavir/ritonavir, lopinavir/ritonavir, itraconazole, voriconazole, mifepristone, mibefradil, LCL161, clarithromycin, posaconazole, telithromycin, grapefruit juice, ceritinib, conivapatan, nefazodone, nelfinavir, saquinavir, ribociclib, idelisib, boceprevir, and atazanavir.

Non-limiting examples of a strong CYP3A4 inducers include, but are not limited to, rifampin, mitotane, avasimibe, rifapentine, apalutamide, ivosidenib, phenytoin, carbamazpeine, enzalutamide, St. John's Wort extract, lumacaftor, and phenobarbital.

Non-limiting examples of a moderate CYP3A4 inducers are ritonavir and St. John's wort, semagacestat, efavirenz, tipranavir and ritonavir, dabrafenib, lesinurad, bosentan, genistein, thioridazine, rifabutin, lorlatinib, nafcillin, lopinavir, daclatasvir and asunaprevir and beclabuvir, modafinil, PF-06282999, etravirine, elagolix, lersirvine, and teleotristat ethyl.

### Dosage Forms of Voxelotor

Voxelotor may be administered in any suitable dosage form, including an oral dosage form.

In some embodiments, voxelotor is administered as a capsule. Capsules of voxelotor are described in U.S. Patent Publication No. 2017/0157101.

In some embodiments, voxelotor is administered as a tablet. Tablets and dispersible tablets of voxelotor are described in U.S. Patent Publication No. US 2018/0125789.

In some embodiments, the tablets described herein comprise voxelotor as a substantially pure crystalline ansolvate form characterized by at least two X-ray powder diffraction peaks (Cu Kα radiation) selected from 13.37°, 14.37°, 19.95° and 23.92°2θ (each ±0.2 °2θ). In some embodiments, the tablets described herein consists essentially of voxelotor as a crystalline ansolvate form characterized by at least two X-ray powder diffraction peaks (Cu Kα radiation) selected from 13.37°, 14.37°, 19.95° and 23.92°2θ (each ±0.2 °2θ). In some embodiments, the tablets described herein comprise a crystalline ansolvate form of voxelotor characterized by X-ray powder diffraction peaks (Cu Kα radiation) at 13.37°, 14.37°, 19.95° and 23.92°2θ (each ±0.2 °2θ).

The tablets described herein comprise voxelotor (e.g., Form I, Form II, or Form N) at an amount of, or at an amount of about, 55%, 56%, 57%, 58%, 59.1%, 59.2%, 59.3%, 59.4%, 59.5%, 59.6%, 59.7%, 59.8%, 59.9%, 60%, 61%, 62%, 63%, 64%, 65% w/w, or a range between any two of these values, wherein the percentage by weight is relative to the total weight of the tablet. In some embodiments, the tablet comprises, or comprises about, 50% to about 70% w/w of voxelotor (e.g., Form II). In some embodiments, the tablet comprises, or comprises about 60% w/w of voxelotor (e.g., Form II).

The tablets described herein comprise about 300 mg to about 1500 mg or about 300 mg to about 900 mg of voxelotor (e.g., Form I, Form II, or Form N). The tablets described herein comprise about 100 mg to about 600 mg of voxelotor (e.g., Form I, Form II, or Form N). In some embodiments, the tablets described herein comprise voxelotor (e.g., Form I, Form II, or Form N) in an amount of, or in an amount of about 500 mg.

The tablets disclosed herein comprise excipients such as a pharmaceutically acceptable filler (also known as diluent), disintegrant, lubricant, surfactant (also known as wetting agent), glidant, and binder.

In some embodiments, the tablets described herein comprise microcrystalline cellulose (MCC). In some embodiments, the MCC is present in the tablet at, or at about, 30%, 31%, 32%, 33%, 34%, 35%, 36%, 37%, 38%, 39%, 40%, or a range between any two of these values, w/w. In some embodiments, the MCC is present in the tablet at about 30% to about 40% w/w. In some embodiments, the MCC is present in the tablet at, or at about, 35% w/w.

The tablets described herein comprise, in some embodiments, one or more disintegrants. Suitable disintegrants include, either individually or in combination, starches including pregelatinized starch and sodium starch glycolate; clays; magnesium aluminum silicate; cellulose-based disintegrants such as powdered cellulose, microcrystalline cellulose, methylcellulose, low-substituted hydroxypropylcellulose, carmellose, carmellose calcium, carmellose sodium and croscarmellose sodium; alginates; povidone; crospovidone; polacrilin potassium; gums such as agar, guar, locust bean, karaya, pectin and tragacanth gums; colloidal silicon dioxide; and the like.

In some embodiments, the disintegrant is croscarmellose sodium. The tablets comprise, or comprise about, 0.5%, 0.55%, 0.6%, 0.65%, 0.7%, 0.75%, 0.8%, 0.85%, 0.9%, 0.95%, 1%, 1.1%, 1.2%, 1.3%, 1.4%, 1.5% 1.6%, 1.7%, 1.8%, 1.9%, or 2% or a range between any two of these values, w/w of one or more disintegrants. In some embodiments, the tablets comprise about 0.25% to about 3% w/w of a disintegrant. In some embodiments, the tablets comprise about 0.25% to about 3% w/w of croscarmellose sodium. In some embodiments, the tablets comprise about 1.25% w/w of croscarmellose sodium.

The tablets described herein comprise one or more surfactants (also known as wetting agents). In some embodiments, the tablets comprise sodium lauryl sulfate as a surfactant. In some embodiments, the surfactant can be present in the tablets disclosed herein at an amount of, or at an amount of about, 0.1%, 0.5%, 1%, 1.1%, 1.2%, 1.3%, 1.4%, 1.45%, 1.5%, 1.55%, 1.6%, 1.65%, 1.7%, 1.75%, 1.8%, 1.9%, 2.0%, 2.5%, 3%, 4%, 5%, or a range between any two of these values, w/w. In some embodiments, the tablets do not comprises a surfactant. In some embodiments, the tablets comprise about 0.5% to about 2.5% w/w of sodium lauryl sulfate. In some embodiments, the tablets comprise sodium lauryl sulfate at an amount of about 1.5% w/w.

The tablets described herein comprise one or more lubricants. Exemplary lubricants include, either individually or in combination, glyceryl behenate; stearic acid and salts thereof, including magnesium, calcium and sodium stearates; hydrogenated vegetable oils; glyceryl palmitostearate; talc; waxes; sodium benzoate; sodium acetate; sodium fumarate; sodium stearyl fumarate; PEGs *(e.g.,* PEG 4000 and PEG 6000); poloxamers; polyvinyl alcohol; sodium oleate; sodium lauryl sulfate; magnesium lauryl sulfate; and the like. In some embodiments, the lubricant can be present in the tablets at an amount of, or at an amount of about, 0.25%, 0.5%, 0.75%, 1%, 1.25%, 1.5%, 1.75%, 2%, 2.5%, 2.75%, 3%, 4%, 5%, or a range between any two of these values, w/w. In some embodiments, the lubricant is magnesium stearate. In some embodiments, magnesium stearate is present in the tablets in the amount of from about 1% to about 5% w/w. In some embodiments, magnesium stearate is present in the tablets in the amount of about 2% w/w.

The tablets described herein comprise one or more binders. Exemplary binding agents and adhesives include, acacia; tragacanth; glucose; polydextrose; starch including pregelatinized starch; gelatin; modified celluloses including methylcellulose, carmellose sodium, hydroxypropylmethylcellulose (HPMC or hypromellose), hydroxypropyl-cellulose, hydroxyethylcellulose and ethylcellulose; dextrins including maltodextrin; zein; alginic acid and salts of alginic acid, for example sodium alginate; magnesium aluminum silicate; bentonite; polyethylene glycol (PEG); polyethylene oxide; guar gum; polysaccharide acids; and the like.

The tablets described herein comprise one or more glidants. Exemplary glidants include, colloidal silicon dioxide, starch, powdered cellulose, sodium lauryl sulfate, magnesium trisilicate and metallic stearates. In some embodiments, the glidant is colloidal silicon dioxide. In some embodiments, the tablet comprises about 0.1% to about 5% by weight of colloidal silicon. In some embodiments, the tablet comprises about 0.5% to about 1% by weight of colloidal silicon dioxide. In some embodiments, the tablet comprises about 0.75% by weight of colloidal silicon dioxide.

Other excipients such as colorants (coloring agents), coating polymers, flavors (flavoring agents), and sweeteners are known in the pharmaceutical art and can be used in the tablets disclosed herein. Non-limiting examples of a sweetener are sucrose, xylitol, maltitol, mannitol, sorbitol, sucralose, sodium saccharin, acesulfame potassium, aspartame, and others known to those of skill in the art. In some embodiments, the coloring agent is iron oxide yellow.

In some embodiments, a tablet comprises about 50% to about 70% by weight voxelotor; about 30% to about 40% by weight of microcrystalline cellulose; about 0.25% to about 3% by weight of croscarmellose sodium; about 1% to about 5% by weight of magnesium stearate; about 0.5% to about 2.5% of sodium lauryl sulfate; and about 0.25% to about 5% by weight of colloidal silicon dioxide; wherein the percentage by weight is relative to the total weight of the tablet.

In some embodiments, tablets described herein include a coating surrounding the core described herein comprising Compound 1. Tablets can be coated using formulations known in the art, such as for example, excipients such as talc, polyvinyl alcohol, and PEG *(e.g.,* PEG 4000 and PEG 6000). In some embodiments, the coating polymer can be hydroxypropyl methylcellulose (HPMC). When coated, tablets comprise a core that is coated with a nonfunctional film or a release-modifying or enteric coating.

### EXAMPLES

The present invention relates to voxelotor for use in a method of treating sickle cell disease in a patient in need thereof wherein the patient has severe hepatic impairment. Examples and data therein concerning patients without severe hepatic impairment are included for reference purposes only.

### Example 1: Characterization of pharmacokinetics and safety of a single oral dose of voxelotor in subjects with hepatic impairment

A Phase 1, open-label study was performed to characterize pharmacokinetics (PK) and safety of a single oral dose of voxelotor in subjects with hepatic impairment. The study was to assess the effects of mild, moderate, or severe hepatic impairment on the PK of a single oral dose of voxelotor, to evaluate the safety and tolerability of a single oral dose of voxelotor in subjects with mild, moderate, or severe hepatic impairment, as well as to establish potential dose adjustment guidance based upon the degree of hepatic insufficiency.

### List of Abbreviations:

| | |
|---|---|
| AE | adverse event |
| BMI | body mass index |
| CYP | Cytochrome P450 |
| CRU | clinical research unit |
| ECG | electrocardiogram |
| eCRF | Electronic case report form |
| LS | least squares |
| max | maximum |
| OTC | over-the-counter |
| PK | pharmacokinetic(s) |
| PT | preferred term |
| SAE | serious adverse event |
| TEAE | treatment-emergent adverse event |

### Study design

This was a Phase 1, multiple-center, nonrandomized, open-label, parallel group study of a single oral dose of voxelotor administered in subjects with mild (Child-Pugh A; score 5 to 6 points), moderate (Child-Pugh B; score 7 to 9 points), or severe (Child-Pugh C; score 10 to 15 points) hepatic impairment and subjects with normal hepatic function. This study was designed to assess the effects of mild, moderate, or severe hepatic impairment on the PK of a single oral dose of voxelotor. Routine safety assessments (AE monitoring, vital signs, physical examinations, clinical laboratory tests, and ECGs) were performed.

Twenty-eight subjects were enrolled (a ratio of 7:7:7:7 subjects per hepatic function group: normal hepatic function, mild, moderate, or severe hepatic impairment, respectively). An additional subject was dosed in error and was discontinued from the study. The group demographics between the normal hepatic function group and the mild, moderate, and severe hepatic impairment groups were similar *(i.e.,* with respect to age [± 10 years], gender, and BMI [± 10%]).

The screening window was 28 days in duration.

Subjects with mild and moderate hepatic impairment were enrolled first. Once at least 4 subjects in each of these 2 groups completed the clinical portion of the study (through Day 5 PK sample), an interim analysis was performed on voxelotor whole blood and plasma concentration data for subjects in these 2 groups comparing data to historical data in healthy subjects to determine if there was an increase in exposure and if a dose adjustment was therefore needed for subjects with severe hepatic impairment. The subjects with normal hepatic function were enrolled last, once at least 4 subjects with severe hepatic impairment were enrolled.

Subjects remained in the CRU until discharge on Day 5 and returned to the CRU on Days 12 (± 1 day) and 20 (± 1 day) for voxelotor whole blood and plasma PK and safety assessments. All subjects (including the subject who terminated the study early) returned for a follow-up visit on Day 28 (± 2 days).

Safety and PK assessments were performed at select time points throughout the study.

### Treatments

Eligible subjects received a single oral dose of voxelotor 1500 mg (5 × 300-mg capsules) on Day 1 (for normal hepatic function, mild and moderate hepatic impairment). A lower dose of voxelotor 600 mg (2 × 300-mg capsules) was administered for the subjects with severe hepatic impairment. The decision to proceed with a lower dose of voxelotor in subjects with severe hepatic impairment was based on the observation of TEAE of diarrhea (mild) occurring in 5 of 7 subjects (71.4%) with moderate hepatic impairment.

Following an overnight fast of at least 10 hours voxelotor was administered with approximately 240 mL (8 fluid ounces) of nonrefrigerated, noncarbonated water. No food was allowed for at least 4 hours postdose. No water or any type of liquid was allowed for 1 hour before dosing through 1-hour postdose with the exception of water consumed for dosing.

For subjects with normal hepatic function, no concomitant medications (prescription, OTC, and herbal, including any drugs that induced study drug specific CYPs) were administered during the study to subjects with normal hepatic function unless they were prescribed by the investigator for treatment of specific clinical events (i.e., AEs).

For hepatically impaired subjects, they were on a stable dose of medication and/or treatment regimen at least 2 weeks prior to study drug dosing (if applicable). Required concomitant medications were administered at least 2 hours before or 8 hours after voxelotor dosing. No herbal medications or strong inducers/inhibitors of CYPs (2B6, 2C9, 2C19, 3A4, and 3A5), or any drug that could have potentially affected the absorption of voxelotor, were administered during the study. No other medication other than already stable doses that the subjects were on was administered during the study unless they were prescribed by the investigator for treatment of specific clinical events *(i.e.,* AEs).

All medications (prescription and OTC), vitamin and mineral supplements, and herbs taken during the study were documented on the concomitant medication eCRF. Information recorded included start and stop dates, dose and route of administration, and indication. Medications taken for a procedure were included.

### Pharmacokinetic and safety variables

Pharmacokinetic variables measured included: Cₘₐₓ, tₘₐₓ, AUCₜ, AUC0-96, AUC_{inf}, t_{½}, λ_{z}, CL/F, V_{z}/F, Fᵤ, AUC_{inf,u}, AUC_{t,u}, C_{max,u}, and CL/Fᵤ.

Adverse events and concomitant medication use were monitored throughout the study. Safety assessments, including physical examinations, vital signs assessments, 12-lead ECGs, and clinical laboratory tests, were performed. Whole blood and plasma concentrations of voxelotor were determined using validated assays.

Analysis populations: (1) "Pharmacokinetic Full Population" includes all subjects who received at least one dose (1500 mg or 600 mg) of voxelotor and had at least one whole blood or plasma concentration data point. (2) "Pharmacokinetic Evaluable Population" includes all subjects who received at least 1 dose of voxelotor and had a sufficient PK profile to derive at least 1 PK parameter. (3) "Safety Population" includes all subjects who received any amount of voxelotor.

Pharmacokinetic variables were calculated from the whole blood and plasma concentrations of voxelotor using noncompartmental methods (Phoenix WinNonlin^{®}, Version 6.3.0395, Pharsight Corp, St. Louis, MO) and actual sampling times. The following PK parameters were determined.

| | |
|---|---|
| AUC₀₋₉₆ | Area under the concentration-time curve from time 0 to 96 hours; calculated using the linear/log trapezoid rule |
| AUC_{inf} | Area under the concentration-time curve from time 0 extrapolated to infinity; calculated as AUCₜ + Cₜ/λ_{z}, where Cₜ was the last quantifiable concentration and λ_{z}. was the terminal elimination rate constant determined by the slope of the terminal phase of the concentration-time curve |
| AUC_{inf,u} | Unbound AUC_{inf} for plasma |
| AUCₜ | Area under the concentration-time curve from time 0 to the time of the last quantifiable concentration; calculated using the linear/log trapezoid rule |
| AUC_{t,u} | Unbound AUCₜ for plasma |
| CL/F | Apparent oral clearance |
| CL/F_{,u} | Unbound CL/F for plasma |
| Cₘₐₓ | Maximum observed concentration |
| C_{max,u} | Unbound Cₘₐₓ for plasma |
| Fᵤ | Fraction of drug unbound in plasma |
| λ_{z} | Terminal elimination rate constant |
| t_{½} | Terminal elimination half-life; calculated as ln(2)/λ_{z} |
| tₘₐₓ | The time that Cmax was observed |
| V_{z}/F | Apparent volume of distribution during the terminal phase |

### Statistical analysis for pharmacokinetic data and safety data

Only data points that described the terminal elimination log-linear decline were used in the regression equation for calculation of λ_{z}; Cₘₐₓ and any data point in the distribution phase were not included in the calculation. A minimum of 3 points was used for determination of λ_{z}. A general rule of R² > 0.80 was considered acceptable for calculation of λ_{z}. If R2 fell below 0.80, then λ_{z} was reported as ND and that subject's λ_{z}, t_{1/2}, and AUC_{inf} were reported as ND in the appropriate listings. If the extrapolated AUC_{inf} was more than 20%, then AUC_{inf} was listed but excluded from descriptive summaries and statistical analysis.

The PK full population was used for all listings. The evaluable PK population was used for summary statistics and statistical analyses. The individual and mean whole blood and plasma drug concentrations of voxelotor versus time curves were presented for subjects. Summary statistics (n, mean, SD, minimum, maximum, and coefficient of variation) were calculated for whole blood drug concentrations and plasma drug concentrations for each time point for voxelotor.

Whole blood and plasma PK parameters were listed for voxelotor. All PK parameters (primary and secondary) were listed. Summary statistics (n, mean, SD, geometric mean, median, minimum, maximum, and coefficient of variation) were calculated for the whole blood and plasma voxelotor PK parameters. For tₘₐₓ, only n, minimum, median and maximum were reported.

To assess the effect of hepatic impairment on the PK of voxelotor, a linear mixed-effect model was fitted to the log-transformed values of whole blood and plasma Cₘₐₓ, AUCₜ, and AUC_{inf}. The model included hepatic impairment group as a fixed effect and age and weight as covariates. Point estimates and 90% CIs for hepatic function group differences (mild, moderate, and severe hepatically-impaired subjects [test] versus the normal hepatic function subjects [control]) on the log scale were exponentiated to obtain estimates for GM ratios on the original scale.

The individual treatment ratio (hepatic versus normal) for the primary PK parameters (Cₘₐₓ, AUCₜ, and AUC_{inf}) along with geometric mean ratio and 90% CI wire displayed.

All statistical analyses were performed using the safety population. All AEs were coded to SOC and PT using MedDRA (Version 19.0) and presented by subject in data listings. A treatment-emergent AE was defined as an AE that was not present prior to treatment with study drug, but appeared following treatment or was present at treatment initiation but worsened during treatment. An AE that was present at treatment initiation but resolved and then reappeared while the subject was on treatment was a TEAE (regardless of the intensity of the AE when the treatment was initiated). Programmatically, an AE was classified as a TEAE if the start date and time occurred on or after the start date and time of first study drug dosing. The overall incidence of TEAEs (number and percentage of subjects) as well as the number of events were summarized by hepatic function group, severity, SAEs, causally related TEAE and SAEs, TEAEs leading to study or treatment discontinuation, life-threatening SAEs, and SAEs resulting in death.

The TEAEs were summarized and tabulated at both the subject (number [%] of subjects) and event (number of events) level: (1) by hepatic function group, SOC, and PT; (2) by hepatic function group, SOC, PT, and maximum reported severity; and (3) by hepatic function group, SOC, PT, and maximum relationship to study drug.

### Baseline characteristics

Physical measurements were performed and the baseline physical measurements are shown in Table A.

**Table A. Summary of baseline physical measurements (safety population)**

| **Parameter** | **Mild (N=7)** | **Moderate (N=7)** | **Severe (N=7)** | **Normal (N=7)** | **Overall (N=28)^{a}** |
|---|---|---|---|---|---|
| Weight (kg) | | | | | |
| Mean | 83.14 | 90.69 | 82.36 | 86.77 | 85.74 |
| SD | 28.285 | 14.258 | 10.457 | 10.526 | 16.829 |
| Median | 76.3 | 94.2 | 84.20 | 91.50 | 89.00 |
| Minimum | 61.8 | 66.2 | 67.7 | 69.7 | 61.8 |
| Maximum | 142.7 | 102.6 | 97.1 | 97.2 | 142.7 |

| Height (cm) | | | | | |
|---|---|---|---|---|---|
| Mean | 172.81 | 167.33 | 168.54 | 173.84 | 170.63 |
| SD | 9.738 | 7.096 | 12.578 | 7.505 | 9.371 |
| Median | 168.8 | 169.00 | 170.00 | 176.50 | 170.00 |
| Minimum | 167.0 | 152.5 | 152.0 | 160.5 | 152.0 |
| Maximum | 194.0 | 174.0 | 185.0 | 180.5 | 194.0 |

| Body Mass Index (kg/m2) | | | | | |
|---|---|---|---|---|---|
| Mean | 27.39 | 32.21 | 29.50 | 28.6 | 29.43 |
| SD | 6.226 | 3.329 | 6.374 | 1.656 | 4.898 |
| Median | 26.40 | 33.00 | 27.00 | 28.20 | 28.65 |
| Minimum | 20.2 | 27.3 | 22.6 | 26.6 | 20.2 |
| Maximum | 37.9 | 36.9 | 38.7 | 31.6 | 38.7 |
| a. An subject with moderate hepatic impairment was dosed in error and was discontinued from the study per sponsor request. The subject was included and listed under the Safety Population; however, has been excluded from table summaries due to the protocol deviation. | | | | | |

### Pharmacokinetic evaluation

Voxelotor whole blood and plasma concentrations were measured using a validated liquid chromatography-mass spectrometry method. The lower limit of assay quantitation for voxelotor was 10.0 ng/mL for whole blood and 5.00 ng/mL in plasma.

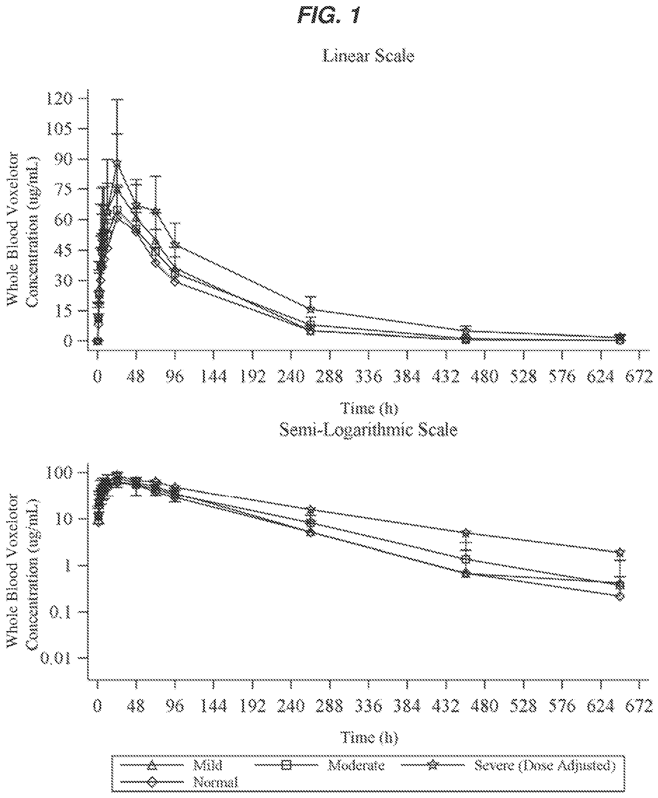

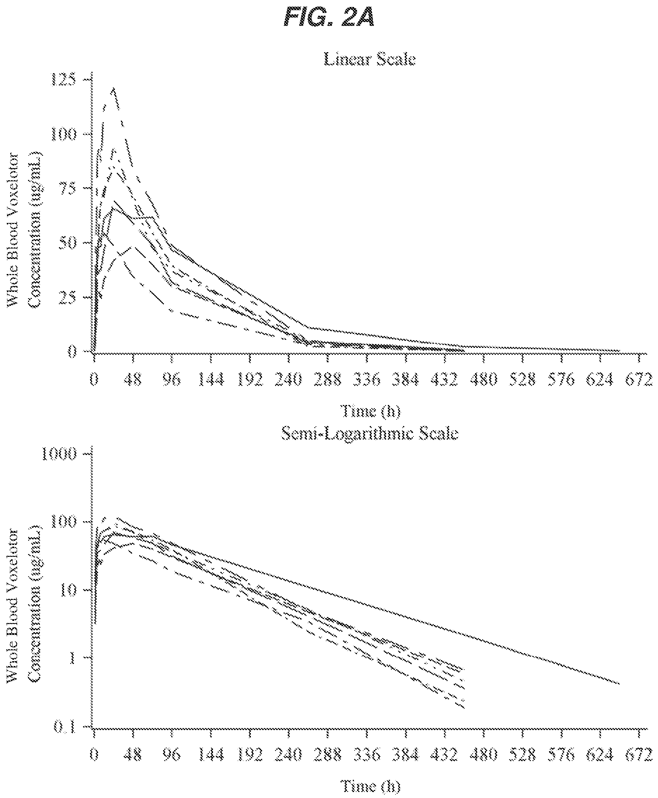

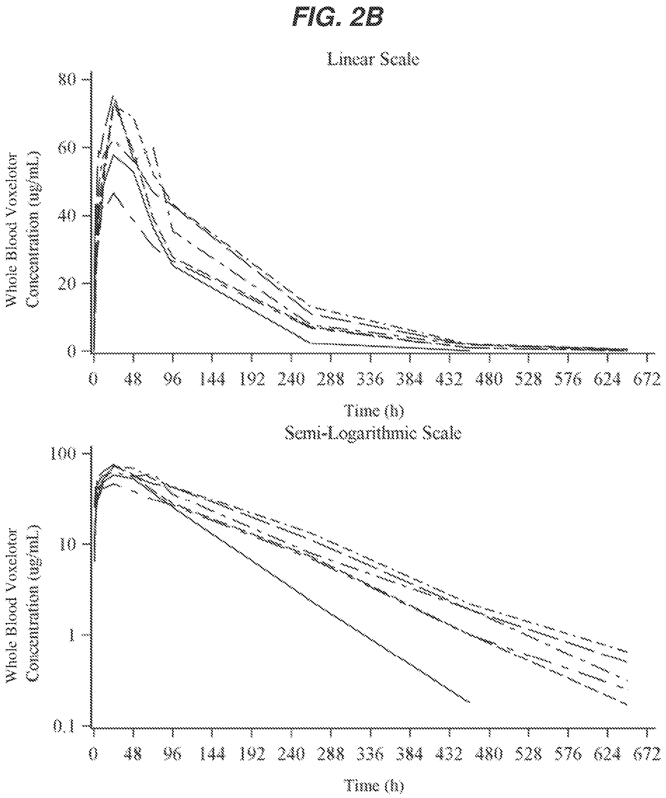

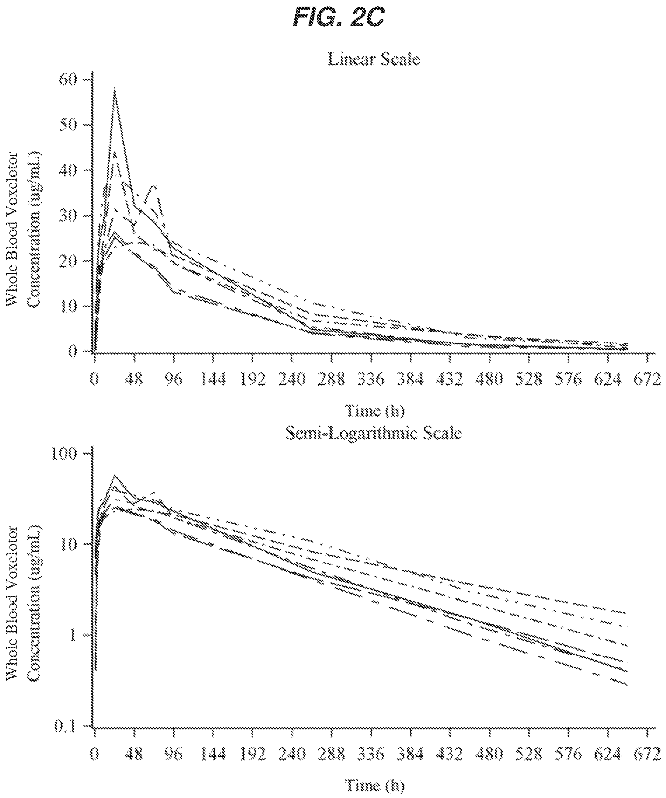

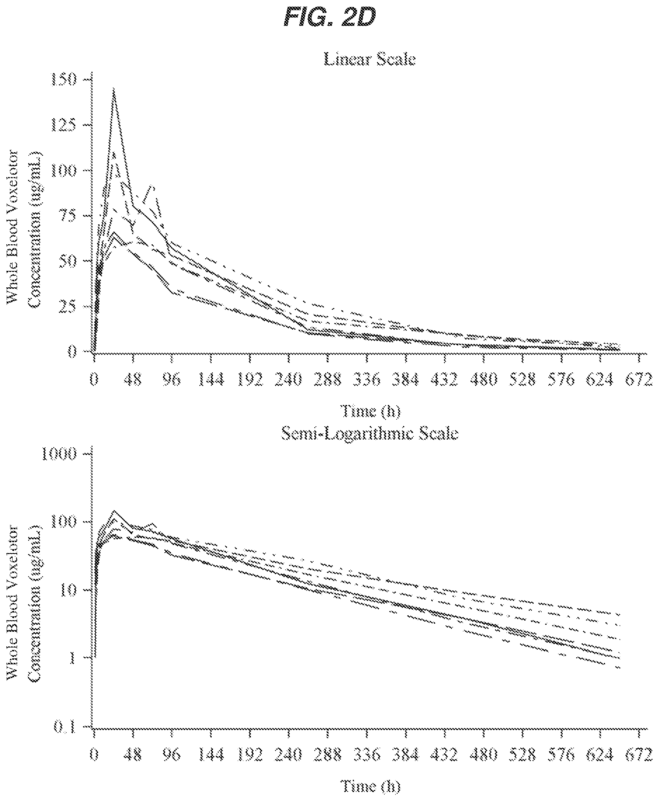

Mean (+ SD) whole blood voxelotor concentration-time profiles are presented in FIG. 1. For subjects with severe hepatic impairment, concentrations were adjusted for dose. Dose adjusted concentration = (concentration/600 mg) x 1500 mg. Overlays of whole blood voxelotor concentration-time profiles are presented in FIGS. 2A-2D with the hepatic impairments status of subjects being mild (FIG. 2A), moderate (FIG. 2B), severe (FIG. 2C), and severe with dose adjusted (FIG. 2D). For subjects with severe hepatic impairment, dose adjustment being done as they were dosed with 600 mg voxelotor compared to 1500 mg for Mild, Moderate and Normal subjects. One subject with moderate hepatic impairment had no measurable voxelotor concentrations in whole blood, but was dosed. This subject was excluded from concentration and PK summaries and statistical analysis.

As shown in FIG. 1, the mean dose adjusted whole blood concentrations were higher in subjects with severe hepatic impairment than in subjects with normal hepatic function. Subjects with mild and moderate hepatic impairment had similar voxelotor concentrations in whole blood to subjects with normal hepatic function.

Individual whole blood voxelotor PK parameters are summarized in Table B. Statistical analyses of the effect of hepatic impairment on voxelotor whole blood PK parameters are summarized in Table C.

**Table B. Summary of whole blood voxelotor pharmacokinetic parameters (pharmacokinetic evaluable population)**

| **Parameter** | **Statistic** | **Mild** | **Moderate** | **Severe^{a}** | **Normal** |
|---|---|---|---|---|---|
| **Cₘₐₓ** (µg/mL) | n | 7 | 6 | 7 | 7 |
| | GM (CV%) | 73.6 (32.8) | 63.8 (17.4) | 86.6 (33.6) | 60.9 (22.4) |
| **AUCₜ** (h* µg/mL) | n | 7 | 6 | 7 | 7 |
| | GM (CV%) | 8193 (29.2) | 8324 (22.6) | 13331 (23.3) | 6953 (10.8) |
| **AUC_{inf}** (h*µg/mL) | n | 7 | 6 | 7 | 7 |
| | GM (CV%) | 8230 (29.1) | 8363 (22.8) | 13636 (24.2) | 6980 (10.9) |
| **AUC₀₋₉₆** (h* µg/mL) | n | 7 | 6 | 7 | 7 |
| | GM (CV%) | 5159 (29.2) | 4656 (15.7) | 6052 (21.4) | 4269 (13.9) |
| **tₘₐₓ** (h) | n | 7 | 6 | 7 | 7 |
| | Median (min, max) | 24.0 (12.0, 48.0) | 24.0 (24.0, 24.0) | 24.0 (24.0, 72.0) | 24.0 (24.0, 48.0) |
| **t_{1/2}** (h) | n | 7 | 6 | 7 | 7 |
| | Mean (SD) | 60.1 (60.7) | 79.5 (18.7) | 111 (19.1) | 63.2 (17.5) |
| **CL/F** (L/h) | n | 7 | 6 | 7 | 7 |
| | GM (CV%) | 0.182 (32.4) | 0.179 (23.9) | 0.110 (26.1) | 0.215 (11.1) |
| **Vz/F** (L) | n | 7 | 6 | 7 | 7 |
| | GM (CV%) | 15.8 (31.0) | 20.6 (18.5) | 17.6 (22.0) | 19.6 (16.4) |
| a. For subjects with severe hepatic impairment AUC and Cₘₐₓ were adjusted for dose. | | | | | |
| Dose adjusted parameter = (parameter/600 mg) × 1500 mg. | | | | | |

**Table C. Statistical analysis of the effect of hepatic impairment on voxelotor pharmacokinetic parameters in whole blood (pharmacokinetic evaluable population)**

| **PK Parameter** | **Comparison** | **No. of Subjects** | | **Geometric LS Mean** | | **Ratio of Geometric LS Mean (Test to Reference)** | **90% CI for Geometric LS Mean Ratio of (Test to Reference)** |
|---|---|---|---|---|---|---|---|
| | | Test | Reference | Test | Reference | | |
| **Cₘₐₓ** (µg/mL) | Mild (Test) vs Normal (Ref) | 7 | 7 | 72.9 | 61.4 | 1.187 | (0.920, 1.530) |
| | Moderate (Test) vs Normal (Ref) | 6 | 7 | 64.9 | 61.4 | 1.056 | (0.796, 1.400) |
| | Severe^{a} (Test) vs Normal (Ref) | 7 | 7 | 85.4 | 61.4 | 1.391 | (1.080, 1.790) |
| **AUCₜ** (h*µg/mL) | Mild (Test) vs Normal (Ref) | 7 | 7 | 8124 | 7127 | 1.140 | (0.922, 1.409) |
| | Moderate (Test) vs Normal (Ref) | 6 | 7 | 8189 | 7127 | 1.149 | (0.908, 1.454) |
| | Severe^{a} (Test) vs Normal (Ref) | 7 | 7 | 13303 | 7127 | 1.867 | (1.512, 2.304) |
| **AUC_{inf}** (h*µg/mL) | Mild (Test) vs Normal (Ref) | 7 | 7 | 8161 | 7151 | 1.141 | (0.920, 1.416) |
| | Moderate (Test) vs Normal (Ref) | 6 | 7 | 8231 | 7151 | 1.151 | (0.906, 1.462) |
| | Severe^{a} (Test) vs Normal (Ref) | 7 | 7 | 13605 | 7151 | 1.902 | (1.536, 2.356) |
| The log-transformed PK parameters (Cmax, AUCt, and AUCinf) were analyzed using a linear mixed-effect model, with fixed effect for hepatic impairment group and age and weight as covariates. | | | | | | | |
| a. For subjects with severe hepatic impairment, AUC and Cₘₐₓ were adjusted for dose. | | | | | | | |
| Dose adjusted parameter = (parameter/600 mg) × 1500 mg. | | | | | | | |

In comparison to subjects with normal hepatic function, the whole blood voxelotor exposures in terms of AUC was 14% to 15% higher in subjects with mild and moderate hepatic impairment. In comparison to subjects with normal hepatic function, the whole blood voxelotor exposures in terms of whole blood AUC adjusted for dose was 87% to 90% higher in subjects with severe hepatic impairment. In comparison to subjects with normal hepatic function, the whole blood Cₘₐₓ was 19%, 6%, and 39% higher in the mild, moderate, and severe hepatic impairment subjects, respectively (Cₘₐₓ was adjusted for dose for the severe hepatic impairment group).

The median tₘₐₓ was similar for all hepatic function groups. Subjects with normal hepatic function, mild, and moderate hepatic impairment had a similar mean t_{1/2} and CL/F. In comparison, the t_{1/2} was longer and the CL/F was lower in subjects with severe hepatic impairment.

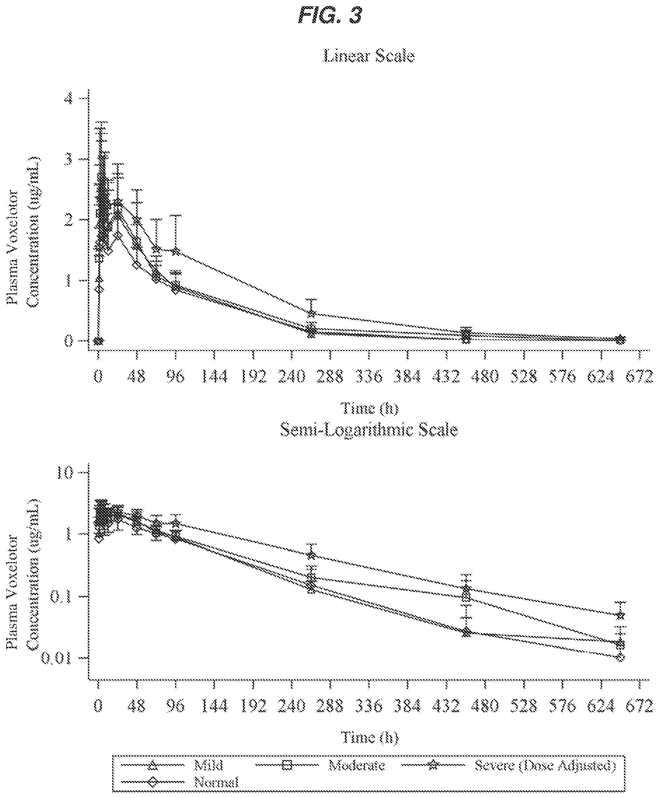

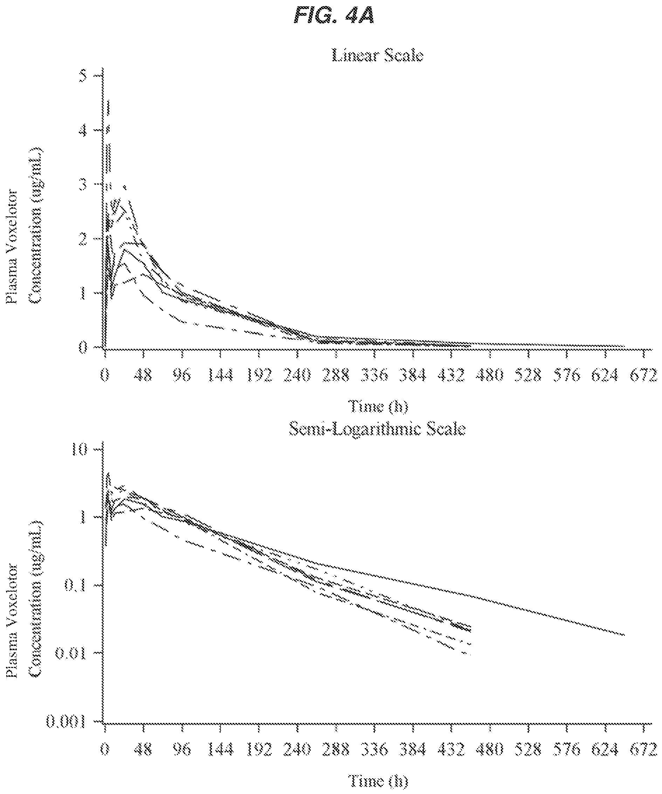

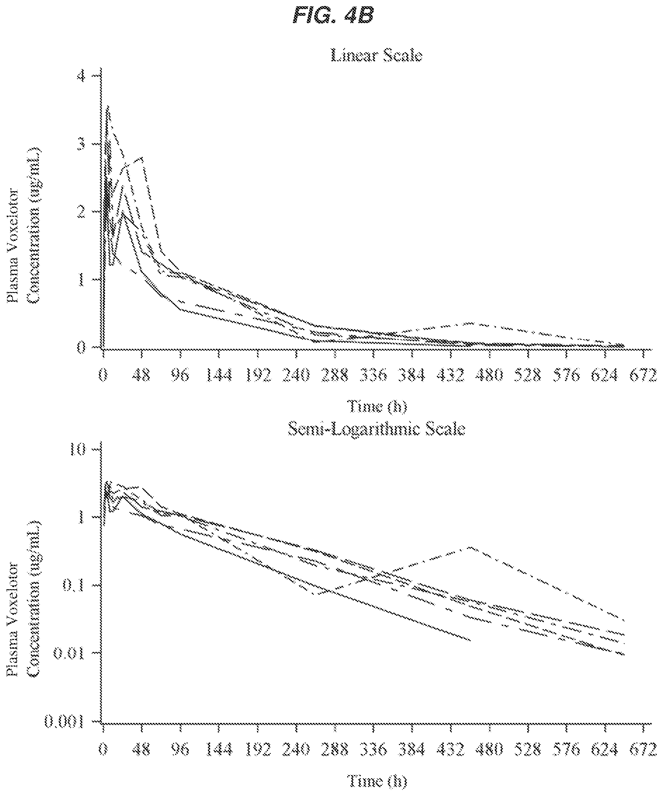

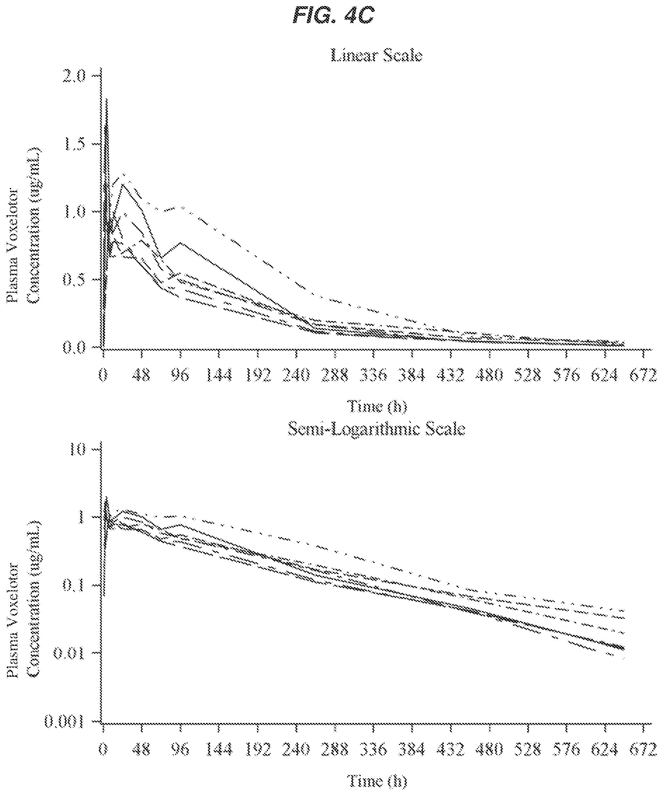

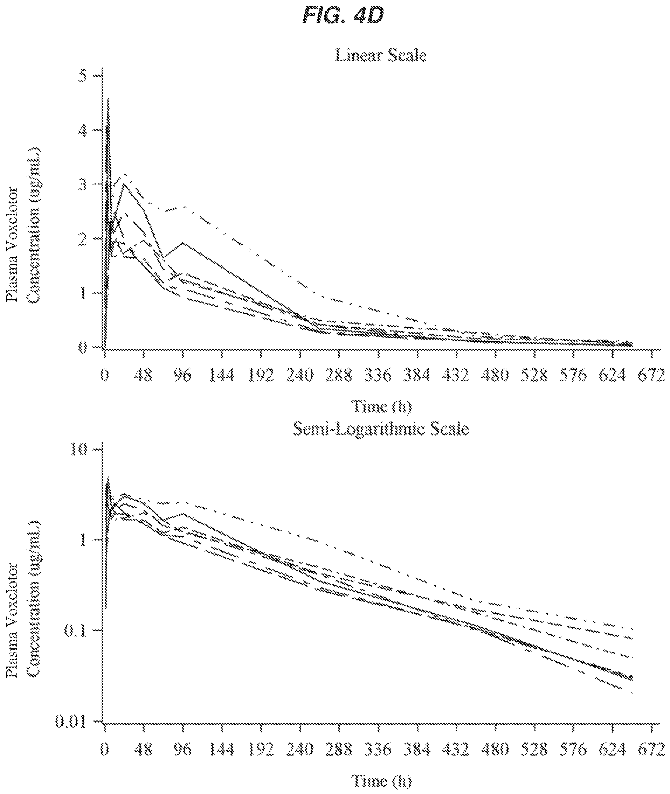

Mean (+ SD) plasma voxelotor concentration-time profiles are presented in FIG. 3. For subjects with severe hepatic impairment, concentrations were adjusted for dose. Dose adjusted concentration = (concentration/600 mg) x 1500 mg. Overlays of plasma voxelotor concentration-time profiles are presented in FIGS. 4A-4D with the hepatic impairments status of subjects being mild (FIG. 4A), moderate (FIG. 4B), severe (FIG. 4C), and severe with dose adjusted (FIG. 4D). For subjects with severe hepatic impairment, dose adjustment being done as they were dosed with 600mg voxelotor compared to 1500mg for Mild, Moderate and Normal subjects. One subject who was dosed 1500 mg in the moderate hepatic impairment group was excluded from concentration summaries, PK summaries, and statistical analysis, due to not having measurable voxelotor concentrations in plasma.

As shown in FIG. 3, the mean dose adjusted plasma concentrations were higher in subjects with severe hepatic impairment than in subjects with normal hepatic function. Subjects with mild and moderate hepatic impairment had similar voxelotor concentrations in plasma to subjects with normal hepatic function.

Individual plasma voxelotor PK parameters and diagnostic results are summarized in Table D. Statistical analysis of the effect of hepatic impairment on voxelotor PK parameters in plasma are summarized in Table E.

**Table D. Summary of plasma voxelotor pharmacokinetic parameters (pharmacokinetic evaluable population)**

| | | **Hepatic Impairment Status** | | | |
|---|---|---|---|---|---|
| **Parameter** | **Statistic** | **Mild** | **Moderate** | **Severe^{a}** | **Normal** |
| **Cₘₐₓ** (µg/mL) | n | 7 | 6 | 7 | 7 |
| | GM (CV%) | 2.39 (39.1) | 2.91 (17.9) | 2.96 (31.1) | 2.01 (23.3) |
| **AUCₜ** (h*µg/mL) | n | 7 | 6 | 7 | 7 |
| | GM (CV%) | 223 (20.0) | 252 (25.3) | 386 (30.4) | 199 (21.2) |
| **AUC_{inf}** (h*µg/mL) | n | 7 | 5 | 7 | 7 |
| | GM (CV%) | 224 (19.9) | 244 (27.4) | 393 (30.8) | 200 (21.1) |
| **AUC₀₋₉₆** (h*µg/mL) | n | 7 | 6 | 7 | 7 |
| | GM (CV%) | 143 (22.2) | 146 (25.5) | 177 (25.5) | 120 (23.0) |
| **tₘₐₓ** (h) | n | 7 | 6 | 7 | 7 |
| | Median (min, max) | 4.00 (2.00, 24.0) | 5.00 (2.00, 48.0) | 4.00 (2.00, 24.0) | 4.00 (2.00, 48.0) |
| **t_{1/2}** (h) | n | 7 | 5 | 7 | 7 |
| | Mean (SD) | 71.4 (17.8) | 90.0 (14.0) | 109 (16.3) | 80.8 (13.0) |
| **CL/F** (L/h) | n | 7 | 5 | 7 | 7 |
| | GM (CV%) | 6.69 (24.4) | 6.14 (28.7) | 3.81 (25.5) | 7.50 (26.0) |
| **Vz/F** (L) | n | 7 | 5 | 7 | 7 |
| | GM (CV%) | 674 (31.4) | 789 (24.3) | 592 (26.6) | 865 (20.2) |
| **Cₘₐₓ Unbound** | n | 7 | 6 | 7 | 7 |
| | GM (CV%) | 0.0593 (51.7) | 0.0106 (50.4) | 0.00622 (63.4) | 0.00817 (29.5) |
| **AUCₜ Unbound** | n | 7 | 6 | 7 | 7 |
| | GM (CV%) | 0.503 (25.2) | 0.919 (48.2) | 2.02 (97.4) | 0.807 (23.1) |
| **AUC_{inf} Unbound** | n | 7 | 5 | 7 | 7 |
| | GM (CV%) | 0.507 (25.1) | 1.04 (42.6) | 2.07 (97.9) | 0.815 (23.1) |
| **CL/F Unbound^{b}** (L/h) | n | 7 | 5 | 7 | 7 |
| | GM (CV%) | 0.0151 (45.0) | 0.0260 (24.3) | 0.0200 (37.6) | 0.0305 (39.1) |
| a. For subjects with severe hepatic impairment AUC and Cₘₐₓ were adjusted for dose. | | | | | |
| b. Result at 4 hours post dose. | | | | | |
| Dose adjusted concentration = (concentration/600 mg) x 1500 mg. | | | | | |

**Table E. Statistical Analysis of the Effect of Hepatic Impairment on Voxelotor Pharmacokinetic Parameters in Plasma (Pharmacokinetic Evaluable Population)**

| | | **Number of Subjects** | | **Geometric LS Mean** | | **Ratio of Geometric LS Mean (Test to Reference)** | **90% CI for Geometric LS Mean Ratio of (Test to Reference)** |
|---|---|---|---|---|---|---|---|
| **PK Paramet er** | **Comparison** | Test | Reference | Test | Reference | | |
| **Cₘₐₓ** (µg/mL) | Mild (Test) vs Normal (Ref) | 7 | 7 | 2.36 | 2.01 | 1.177 | (0.917, 1.510) |
| | Moderate (Test) vs Normal (Ref) | 6 | 7 | 3.03 | 2.01 | 1.510 | (1.145, 1.992) |
| | Severe^{a} (Test) vs Normal (Ref) | 7 | 7 | 2.90 | 2.01 | 1.445 | (1.128, 1.852) |
| **AUCₜ** (h*µg/m L) | Mild (Test) vs Normal (Ref) | 7 | 7 | 221 | 203 | 1.089 | (0.860, 1.379) |
| | Moderate (Test) vs Normal (Ref) | 6 | 7 | 245 | 203 | 1.207 | (0.929, 1.568) |
| | Severe^{a} (Test) vs Normal (Ref) | 7 | 7 | 388 | 203 | 1.907 | (1.508, 2.411) |
| **AUC_{inf}** (h*µg/m L) | Mild (Test) vs Normal (Ref) | 7 | 7 | 223 | 204 | 1.093 | (0.858, 1.392) |
| | Moderate (Test) vs Normal (Ref) | 5 | 7 | 240 | 204 | 1.180 | (0.895, 1.556) |
| | Severe^{a} (Test) vs Normal (Ref) | 7 | 7 | 394 | 204 | 1.932 | (1.521, 2.456) |
| The log-transformed PK parameters (Cₘₐₓ, AUCₜ, and AUC_{inf}) are analyzed using a linear mixed-effect model, with fixed effect for hepatic impairment group and age and weight as covariates. | | | | | | | |
| a. For subjects with severe hepatic impairment AUC and Cₘₐₓ were adjusted for dose. | | | | | | | |
| Dose adjusted concentration = (concentration/600 mg) × 1500 mg. | | | | | | | |

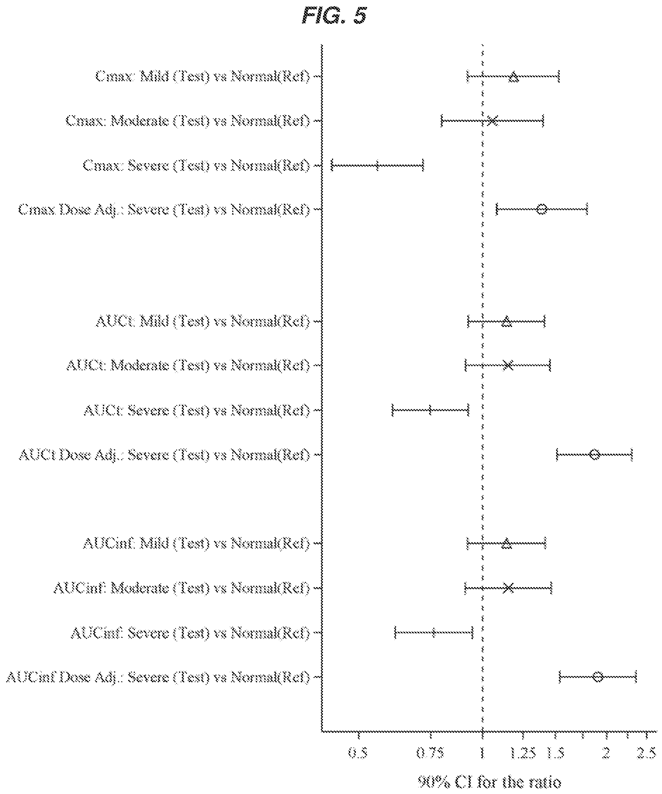

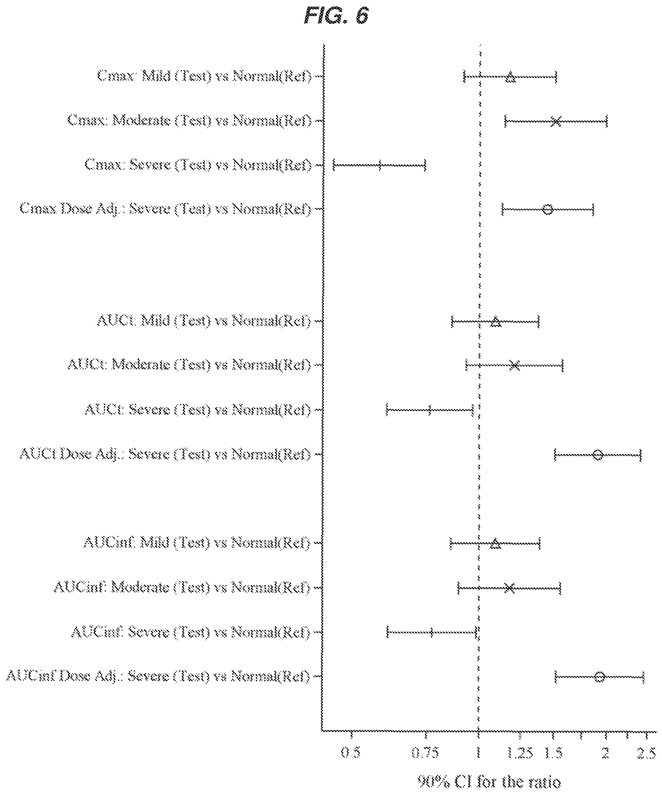

Plots of individual treatment ratios of PK parameters of voxelotor in whole blood and plasma by hepatic impairment status group are presented in FIG. 5 and FIG. 6 respectively. FIGS. 5-6 show the comparisons of Mild vs Normal; Moderate vs Normal; Severe vs Normal; and Severe(Dose Adjusted) vs Normal.

The exposure in terms of AUC for voxelotor in plasma was 9% to 21% higher in subjects with mild and moderate hepatic impairment compared to subjects with normal hepatic function. voxelotor exposures in terms of plasma AUC adjusted for dose was 90% to 93% higher in subjects with severe hepatic impairment compared to subjects with normal hepatic function. In comparison to subjects with normal hepatic function, the Cₘₐₓ was 18%, 51%, and 45% higher in subjects with mild, moderate, and severe hepatic impairment, respectively (Cₘₐₓ was adjusted for dose for the severe hepatic impairment group).

The median tₘₐₓ was similar for all of the hepatic function groups. Subjects with normal hepatic function, mild, and moderate hepatic impairment had a similar t_{1/2} and CL/F. In comparison, the t_{1/2} was longer and the CL/F was lower in subjects with severe hepatic impairment.

The fraction unbound of voxelotor measured at the 4-hour timepoint was similar across all groups. The unbound parameters therefore showed similar results to the bound parameters.

Pharmacokinetic conclusions: Whole blood and plasma voxelotor exposures were 87% to 93% higher in subjects with severe hepatic impairment compared to subjects with normal hepatic function, while subjects with mild to moderate hepatic impairment were only 9% to 21% higher compared to subjects with normal hepatic function. Based on this increase in exposure in subjects with severe hepatic impairment, a dose adjustment is warranted in these subjects. No dose adjustment is warranted in subjects with mild to moderate hepatic impairment.

### Safety evaluation

As shown herein, voxelotor was safe and well tolerated when administered as a single oral dose of 1500 mg to subjects with mild (Child-Pugh A), moderate (Child-Pugh B), and subjects with normal hepatic function, or 600 mg to subjects with severe (Child-Pugh C) hepatic impairment.

No deaths, other serious adverse events (SAEs), and no AEs leading to discontinuation of the study were reported.

Overall, 11 subjects (39.3%) reported 17 TEAEs over the course of the study, among which 15 were considered mild and 2 were considered moderate. The most frequently reported TEAE (in 3 or more subjects overall) was diarrhea (7 events in 7 subjects [25% of subjects]). The 17 TEAEs occurred only in subjects with mild, moderate, or severe hepatic impairment status.

Subjects with mild hepatic impairment: 4 of 7 subjects (57.1%) reported mild TEAEs. A total of 7 mild TEAEs (diarrhea [2 events], dyspepsia [2 events], sinus tachycardia, ligament sprain, and headache) were reported.

Subjects with moderate hepatic impairment: 5 of 7 subjects (71.4%) reported mild TEAEs, and 1 of 7 subjects (14.3%) reported a moderate TEAE. A total of 7 mild TEAEs (diarrhea [5 events], vomiting, and influenza) and 1 moderate TEAE (nephrolithiasis) were reported.

Subjects with severe hepatic impairment: 1 of 7 subjects (14.3%) reported a mild TEAE and a moderate TEAE. A total of 1 mild TEAE (pyrexia) and 1 moderate TEAE (dyspnoea) were reported. Subjects with normal hepatic function: No TEAEs were reported.

Seven subjects (25.0%) reported 8 TEAEs which were considered by the investigator to be possibly/probably related to study drug. These included 6 events of diarrhea, 1 event of dyspepsia, and 1 event of headache.

With the exception of 2 mild TEAEs (headache and dyspepsia) and 1 moderate TEAE (dyspnoea), all other TEAEs resolved without requiring any treatment.

### Example 2 (for reference only)

The effect of voxelotor on the pharmacokinetics (PK) of a single dose of caffeine, warfarin sodium, omeprazole, and midazolam hydrochloride, which are probe substrates for cytochrome P450 (CYP)1A2, CYP2C9, CYP2C19, and CYP3A4, respectively, were studied in healthy subjects. During Period 1, subjects received a single dose of caffeine 100 mg, warfarin sodium 10 mg + vitamin K 10 mg, omeprazole 20 mg, and midazolam hydrochloride 2 mg on Day 1. During Period 2, subjects received voxelotor 900 mg QD on Days 1 and 2 followed by 600 mg QD on Day 3, a single dose of caffeine 100 mg, warfarin sodium 10 mg + vitamin K 10 mg, omeprazole 20 mg, and midazolam hydrochloride 2 mg on Day 4, and voxelotor 600 mg QD on Days 4 through 7.

Whole blood and plasma concentrations of voxelotor and plasma concentrations of caffeine, paraxanthine, S-warfarin, omeprazole, 5-hydroxyomeprazole, midazolam, and 1-hydroxymidazolam were determined using validated assays. Pharmacokinetic variables were calculated from the whole blood and plasma concentrations using noncompartmental methods including: maximum observed plasma concentration (Cₘₐₓ), area under the plasma concentration-time curve (AUC) from time 0 to the time of the last quantifiable concentration (AUCₜ), AUC from time 0 extrapolated to infinity (AUC_{inf}), the time that Cₘₐₓ was observed (tₘₐₓ), and terminal elimination half-life (t_{½}) for caffeine, paraxanthine, S-warfarin, omeprazole, 5-hydroxyomeprazole, midazolam, and 1-hydroxymidazolam; ratio of metabolite to parent Cₘₐₓ, AUCₜ, and AUC_{inf} corrected for molecular weight for paraxanthine/caffeine, 5-hydroxyomeprazole/omeprazole, and 1-hydroxymidazolam/midazolam in plasma; and Cₘₐₓ, tₘₐₓ, AUC from time 0 to 24 hours (Days 4 and 7), and t_{½} (Day 7) for voxelotor in whole blood and plasma.

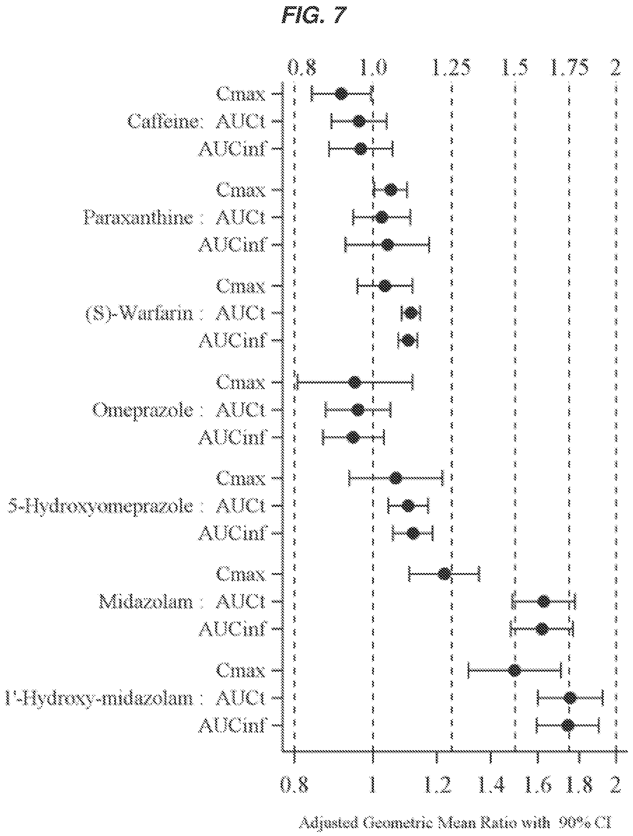

The effect of multiple doses on voxelotor on the PK of probe substrates for CYP1A2 (caffeine), CYP2C9 (S-warfarin), CYP2C19 (omeprazole), and CYP3A4 (midazolam) and metabolites paraxanthine, 5-hydroxyomeprazole, and 1-hydroxymidazolam is presented in Figure 7.

From these studies, it was determined that the administration of voxelotor had no clinically meaningful effect on the PK of probe substrates for CYP1A2 (caffeine), CYP2C9 (S-warfarin), or CYP2C19 (omeprazole). Overall exposure of the CYP3A4 probe substrate (midazolam) was increased by 63% in the presence of voxelotor. The overall exposure of the midazolam metabolite, 1'-hydroxy-midazolam, was increased by 75% in the presence of voxelotor. Whole blood voxelotor exposure was approximately 20 to 25 times higher than plasma voxelotor exposure. Voxelotor was safe and well tolerated when administered alone and in combination with caffeine, warfarin sodium, omeprazole, and midazolam hydrochloride to healthy subjects.

A CYP3A4-mediated time dependent inhibition PBPK model (described in Example 3) was used to simulate the effects of voxelotor on the kinetics of midazolam. Specifically, simulated plasma concentrations of midazolam in the absence and presence of voxelotor (1500 mg QD) in patients with sickle cell disease were performed. The results are shown in Table F.

Based on this data, it is contemplated that voxelotor is a moderate inhibitor of CYP3A4.

**Table F. Simulated Geometric Mean Ratios for Cₘₐₓ and AUC of midazolam in the absence and presence of voxelotor (1500 mg QD) in patients with SCD**

| | Control | | Presence of Voxelotor | | Ratio | |
|---|---|---|---|---|---|---|
| | Cₘₐₓ (ng/mL) | AUC (ng/mL*h) | Cₘₐₓ (ng/mL) | AUC (ng/mL*h) | Cₘₐₓ | AUC |
| Simulated | 7.04 | 22.23 | 14.44 | 69.67 | 2.05 (2.02-2.17) | 3.13 (2.95-3.47) |

### Example 3 (for reference only)

PBPK modeling and simulation was also used to evaluate voxelotor as an object (victim) of drug interactions.

The relative contributions of enzymes to the formation of oxidative, glucuronidation, and reduction products of voxelotor were assigned on the basis of in vitro metabolism data and in vivo mass balance/metabolite profile data from previous studies, and a base model for PK in healthy subjects was developed using previous single-dose PK studies. Model verification was performed using clinical datasets from previous multiple-dose DDI studies in healthy subjects. Such previous studies were carried out according to methods known in the art. Subsequently, the model was refined and adjusted to describe the PK of voxelotor in subjects with sickle cell disease. Literature data on biochemical and physiological parameters likely to be affected by the disease and clinical data were used.

A PBPK model incorporated in a Simcyp simulator that considers both liver and intestinal metabolism was used, as it described the disposition of voxelotor with reasonable accuracy when compared with clinical data. The modeling studies were performed using a dose of 1500 mg of voxelotor.

Results for modeling studies of voxelotor, administered in a single dose, in the absence and presence of CYP3A4 modulators are summarized in Table G. Results for modeling studies of voxelotor, administered in multiple doses, in the absence and presence of CYP3A4 modulators are summarized in Table H. In these tables, "fmCYP3A4" refers to fraction metabolized by CYP3A4.

**Table G. Predicted Geometric Mean (GM) Ratios* for Plasma and Whole Blood Cₘₐₓ and AUC of Voxelotor (Single Dose) in the Absence and Presence of CYP3A4 Modulators (Multiple Dose) in SCD patients**

| **CYP3A4 Modulator** | **Ratio** | |
|---|---|---|
| | GM Cₘₐₓ | GM AUC |
| Ketoconazole (400 mg) | 1.05 | 1.83 |
| Strong CYP3A4 inhibitor | (1.04-1.05) | (1.75-1.91) |
| Fluconazole (400 mg) | 1.05 | 2.16 |
| Moderate CYP3A4 inhibitor | (1.04-1.05) | (2.09-2.24) |
| Fluconazole (200 mg) | 1.04 | 1.84 |
| Moderate CYP3A4 inhibitor | (1.04-1.04) | (1.79-1.89) |
| Fluvoxamine (50 mg) | 1.02 | 1.20 |
| Weak CYP3A4 inhibitor | (1.01-1.02) | (1.19-1.22) |
| Rifampicin (600 mg) | 0.86 | 0.28 |
| Strong CYP3A4 inducer | (0.84-0.87) | (0.26-0.30) |
| Efavirenz (600 mg) | 0.94 | 0.46 |
| Moderate CYP3A4 inducer | (0.93-0.94) | (0.44-0.50) |

| | | |
|---|---|---|
| *Geometric mean ratios for whole blood are identical; assumes fmCYP3A4 0.75. | | |

**Table H. Predicted Geometric Mean (GM) Ratios* for Plasma and Whole Blood Cₘₐₓ and AUC of Voxelotor (Multiple Doses) in the Absence and Presence of CYP3A4 Modulators (Multiple Dose) in SCD patients**

| **CYP3A4 Modulator** | **Ratio** | | |
|---|---|---|---|
| | GM Cₘₐₓ | GM Cₘᵢₙ | GM AUC |
| Ketoconazole (400 mg) | 1.40 | 1.52 | 1.46 |
| Strong CYP3A4 inhibitor | (1.35-1.45) | (1.46-1.59) | (1.46-1.59) |
| Fluconazole (400 mg) | 1.70 | 1.94 | 1.80 |
| Moderate CYP3A4 inhibitor | (1.64-1.77) | (1.85-2.04) | (1.73-1.88) |
| Fluconazole (200 mg) | *1.55* | 1.74 | 1.63 |
| Moderate CYP3A4 inhibitor | (1.51-1.60) | (1.67-1.82) | (1.58-1.69) |
| Fluvoxamine (50 mg) | 1.22 | 1.28 | 1.24 |
| Weak CYP3A4 inhibitor | (1.19-1.24) | (1.25-1.31) | (1.22-1.27) |
| Rifampicin (600 mg) | 0.33 | 0.11 | 0.23 |
| Strong CYP3A4 inducer | (0.31-0.35) | (0.09-0.14) | (0.21-0.25) |
| Efavirenz (600 mg) | 0.48 | 0.31 | 0.40 |
| Moderate CYP3A4 inducer | (0.44-0.51) | (0.28-0.35) | (0.37-0.44) |

| | | | |
|---|---|---|---|
| *Geometric mean ratios for whole blood are identical; assumes fmCYP3A4 0.75. | | | |

Based on the above data, it is contemplated that co-administration of moderate or strong CYP3A4 inducers can decrease voxelotor plasma and whole blood exposures (Cₘᵢₙ, Cₘₐₓ, and AUC), which can thereby reduce the efficacy of voxelotor. It is further contemplated that co-administration of strong CYP3A4 inhibitors can increase plasma and whole blood exposures (Cₘₐₓ and AUC).

### Example 4

### 1 Indications and Usage

OXBRYTA is indicated for the treatment of sickle cell disease (SCD) in adults and pediatric patients 12 years of age and older.

This indication is approved under accelerated approval based on increase in hemoglobin (Hb) *[see Clinical Studies (14)].* Continued approval for this indication may be contingent upon verification and description of clinical benefit in confirmatory trial(s).

### 2 Dosage and Administration

### 2.1 Recommended Dosage for Sickle Cell Disease

The recommended dosage of OXBRYTA is 1,500 mg taken orally once daily with or without food. If a dose is missed, continue dosing on the day following the missed dose.

Patients should swallow OXBRYTA tablets whole. Do not cut, crush, or chew the tablets. OXBRYTA may be given with or without hydroxyurea.

### 2.2 Recommended Dosage for Hepatic Impairment

The recommended dosage of OXBRYTA in patients with severe hepatic impairment (Child Pugh C) is 1,000 mg taken once daily with or without food. No dosage adjustment of OXBRYTA is required for patients with mild or moderate hepatic impairment *[see Use in Specific Populations (8.6) and Clinical Pharmacology (12.3)].*

### 2.3 Recommended Dosage of OXBRYTA When Used with Concomitant Moderate or Strong Inducers, Strong Inhibitors of CYP3A4, or Fluconazole

Avoid concomitant use of strong or moderate CYP3A4 inducers, strong CYP3A4 inhibitors, or fluconazole with OXBRYTA [*see Drug Interactions (7.1) and Clinical Pharmacology (12.3)].* If concomitant use of strong or moderate CYP3A4 inducers, strong CYP3A4 inhibitors, or fluconazole is unavoidable, adjust the OXBRYTA dosage as recommended in Table 1.

**Table 1: OXBRYTA Recommended Dosage for Concomitant Medications**

| **Concomitant Medication** | **Recommended OXBRYTA Dosage** |
|---|---|
| Strong CYP3A4 inhibitors or fluconazole | 1,000 mg once daily |
| Strong or moderate CYP3A4 inducers | 2,500 mg once daily |

### 3 Dosage Forms and Strengths

Tablets: 500 mg light yellow to yellow, oval shaped, biconvex, debossed with "GBT 500" on one side.

### 4 Contraindications

OXBRYTA is contraindicated in patients with a history of serious drug hypersensitivity reaction to voxelotor or excipients. Clinical manifestations may include generalized rash, urticaria, mild shortness of breath, mild facial swelling, and eosinophilia *[see Warnings and Precautions (5.1), and Adverse Reactions (6.1)].*

### 5 Warnings and Precautions

### 5.1 Hypersensitivity Reactions

Serious hypersensitivity reactions after administration of OXBRYTA have occurred in <1% of patients treated. Clinical manifestations may include generalized rash, urticaria, mild shortness of breath, mild facial swelling, and eosinophilia *[see Adverse Reactions (6.1)].*

If hypersensitivity reactions occur, discontinue OXBRYTA and administer appropriate medical therapy. Do not reinitiate OXBRYTA in patients who experience these symptoms with previous use.

### 5.2 Laboratory Test Interference

OXBRYTA administration may interfere with measurement of Hb subtypes (HbA, HbS, and HbF) by high-performance liquid chromatography (HPLC) *[see Drug Interactions (7.3)].* If precise quantitation of Hb species is required, chromatography should be performed when the patient is not receiving OXBRYTA therapy.

### 6 Adverse Reactions

The following clinically significant adverse reaction is discussed in other sections of the labeling: Hypersensitivity Reactions *[see Contraindications (4)].*

### 6.1 Clinical Trials Experience

Because clinical trials are conducted under widely varying conditions, adverse reaction rates observed in the clinical trials of a drug cannot be directly compared to rates in the clinical trials of another drug and may not reflect the rates observed in practice.

The safety of OXBRYTA was evaluated in the HOPE trial based upon 88 patients who received OXBRYTA 1,500 mg and 91 patients who received placebo orally once daily *[see Clinical Studies (14)].* Seventy-four patients received OXBRYTA 1,500 mg once daily for ≥24 weeks and 65 patients for ≥48 weeks.

In patients who received OXBRYTA 1,500 mg once daily the median age was 24 years (range: 12-59); 65% female; 66% Black or African American and 23% Arab/Middle Eastern; and 65% receiving hydroxyurea at baseline.

Serious adverse reactions occurred in 3% (3/88) of patients receiving OXBRYTA 1,500 mg, which included headache, drug hypersensitivity, and pulmonary embolism occurring in 1 patient each. Permanent discontinuation due to an adverse reaction (Grades 1-4) occurred in 5% (4/88) of patients who received OXBRYTA 1,500 mg.

Dosage modifications (dose reduction or dosing interruption) due to an adverse reaction occurred in 41% (36/88) of patients who received OXBRYTA. Most frequent adverse reactions requiring dosage interruption occurring in more than one patient who received OXBRYTA 1,500 mg included diarrhea, headache, rash, and vomiting.

The safety profile observed in pediatric patients 12 to <17 years of age treated with OXBRYTA was similar to that seen in adult patients.

The most common adverse reactions occurring in ≥10% of patients treated with OXBRYTA 1,500 mg with a difference of >3% compared to placebo are summarized in Table 2.

**Table 2: Adverse Reactions (≥10%) in Patients Receiving OXBRYTA with a Difference Between Arms of >3% Compared to Placebo in HOPE**

| **Adverse Reaction ^{a}** | **OXBRYTA 1,500 mg (N=88)** | **Placebo (N=91)** |
|---|---|---|
| Headache | 23 (26%) | 20 (22%) |
| Diarrhea | 18 (20%) | 9 (10%) |
| Abdominal Pain ^{b} | 17 (19%) | 12 (13%) |
| Nausea | 15 (17%) | 9 (10%) |
| Fatigue | 12 (14%) | 9 (10%) |
| Rash ^{c} | 12 (14%) | 9 (10%) |
| Pyrexia | 11 (12%) | 6 (7%) |

| | | |
|---|---|---|
| ^{a} Adverse reactions were Grades 1 or 2 except for Grade 3 diarrhea (1), nausea (1), rash (1), and rash generalized (3) ^{b} Abdominal pain (grouped PTs) included the following PTs: abdominal pain and upper abdominal pain ^{c} Rash (grouped PTs) includes the following PTs: rash, urticaria, generalized rash, maculo-papular rash, pruritic rash, papular rash, erythematous rash, and vesicular rash | | |

Clinically relevant adverse reactions occurring in <10% of patients included:
- Drug hypersensitivity

### 7 Drug Interactions

### 7.1 Effect of Other Drugs on Voxelotor

### Strong CYP3A4 Inhibitors or Fluconazole

Co-administration of strong CYP3A4 inhibitors or fluconazole may increase voxelotor plasma concentrations and may lead to increased toxicity.

Avoid co-administration of OXBRYTA with strong CYP3A4 inhibitors or fluconazole and replace these drugs with alternative drugs when possible *[see Clinical Pharmacology (12.3)].* Decrease the OXBRYTA dosage when co-administration with a strong CYP3A4 inhibitor or fluconazole is unavoidable *[see Dosage and Administration (2.3)].*

### Strong or Moderate CYP3A4 Inducers

Co-administration of strong or moderate CYP3A4 inducers may decrease voxelotor plasma concentrations and may lead to reduced efficacy.

Avoid co-administration of OXBRYTA with strong or moderate CYP3A4 inducers. Increase the OXBRYTA dosage when co-administration with a strong or moderate CYP3A4 inducer is unavoidable *[see Dosage and Administration (2.3)]*

### 7.2 Effect of Voxelotor on Other Drugs

Voxelotor increased the systemic exposure of midazolam (a sensitive CYP3A4 substrate) *[see Clinical Pharmacology (12.3)*]*.* Avoid co-administration of OXBRYTA with sensitive CYP3A4 substrates with a narrow therapeutic index. If concomitant use is unavoidable, consider dose reduction of the sensitive CYP3A4 substrate(s).

### 7.3 Laboratory Test Interference

OXBRYTA administration may interfere with measurement of Hb subtypes (HbA, HbS, and HbF) by HPLC *[see Warnings and Precautions (5.2)*]*.* If precise quantitation of Hb species is required, chromatography should be performed when the patient is not receiving OXBRYTA therapy.

### 8 Use in Specific Populations

### 8.1 Pregnancy

### Risk Summary

There are no available data on OXBRYTA use in pregnant women to evaluate for a drug-associated risk of major birth defects, miscarriage or adverse maternal or fetal outcomes. In animal reproduction studies, oral administration of voxelotor to pregnant rats and rabbits during organogenesis at exposures up to 2.8-times (rats) and 0.3-times (rabbits) the exposure at the maximum recommended human dose resulted in no adverse developmental effects (see *Data*)*.*

The estimated background risk of major birth defects and miscarriage for the indicated population is approximately 14% and up to 43%, respectively. All pregnancies have a background risk of birth defect, loss, or other adverse outcomes.

There are adverse effects on maternal and fetal outcomes associated with sickle cell disease in pregnancy *(see Clinical Considerations).* OXBRYTA should only be used during pregnancy if the benefit of the drug outweighs the potential risk.

### Clinical Considerations

### Disease-Associated Maternal and/or Embryo/Fetal Risk

Women with sickle cell disease have an increased risk of adverse pregnancy outcomes for the mother and the fetus. Pregnant women are at greater risk for vasoocclusive crises, pre-eclampsia, eclampsia, and maternal mortality. For the fetus, there is an increased risk for intrauterine growth restriction, preterm delivery, low birth weight, and perinatal mortality.

### Data

### Animal Data

In embryo-fetal development studies, voxelotor was administered orally to pregnant rats at 15, 50, and 250 mg/kg/day (gestation days 7 through 17) and rabbits at 25, 75, and 150 mg/kg/day (gestation days 7 through 19) through organogenesis. Maternal toxicity was observed at the highest dose levels in these studies equivalent to 2.8-times (rats) and 0.3-times (rabbits) the exposures in patients receiving OXBRYTA at the recommended daily dose. There was no evidence of adverse developmental outcomes in rats or rabbits.

In a pre- and postnatal development study, voxelotor was administered orally to pregnant rats at 15, 50 and 250 mg/kg/day (gestation day 6 through lactation day 20). Maternal gestational body weights were decreased at 250 mg/kg/day, which continued to the end of lactation. The findings in offspring included reduced survival and reduced body weights throughout lactation, weaning and maturation. The effects in offspring were observed at the maternal dose of 250 mg/kg/day with an exposure approximately 2.8-times the exposure in patients at the recommended dose.

### 8.2 Lactation

### Risk Summary

There are no data on the presence of voxelotor in human milk, the effects on the breastfed child, or the effects on milk production. Voxelotor was detected in milk in lactating rats. Plasma concentrations of voxelotor in pregnant rats were higher than the concentration in milk. When a drug is present in animal milk, it is likely that the drug will be present in human milk. The concentration of voxelotor in animal milk does not necessarily predict the concentration of drug in human milk. Because of the potential for serious adverse reactions in the breastfed child, including changes in the hematopoietic system, advise patients that breastfeeding is not recommended during treatment with OXBRYTA, and for at least 2 weeks after the last dose.

### 8.4 Pediatric Use

The safety and effectiveness of OXBRYTA for sickle cell disease have been established in pediatric patients aged 12 years and older. Use of OXBRYTA for sickle cell disease is supported by evidence from an adequate and well-controlled study in adults and pediatric patients (HOPE trial). The HOPE trial enrolled a total of 26 pediatric patients aged 12 to <17 years, in which 12 pediatric patients received OXBRYTA 1,500 mg once daily and 14 pediatric patients received OXBRYTA 900 mg once daily *[see Adverse Reactions (6.1), Clinical Pharmacology (12.3), and Clinical Studies (14)].* The safety and efficacy of OXBRYTA in pediatric patients below the age of 12 years have not been established.

Pharmacokinetics, safety and efficacy in pediatric patients 12 years to <17 years were similar to that observed in adults *[see Dosage and Administration (2), Clinical Pharmacology (12.3) and Clinical Studies (14)].*

The adverse reactions observed in pediatric patients 12 to <17 years treated with OXBRYTA were similar in type and frequency to those observed in adults *[see Adverse Reactions (6.1)].*

### 8.5 Geriatric Use

Clinical studies of OXBRYTA did not include sufficient numbers of subjects aged 65 and over to determine whether they respond differently from younger subjects.

### 8.6 Hepatic Impairment

Severe hepatic impairment increases voxelotor exposures *[see Clinical Pharmacology (12.3)].* Reduce OXBRYTA dose *[see Dosage and Administration (2.2)].*

### 11 Description

Voxelotor is a hemoglobin S polymerization inhibitor. The chemical name of voxelotor is: 2-hydroxy-6-((2-(1-isopropyl-1H-pyrazol-5-yl)pyridin-3-yl)methoxy)benzaldehyde. Voxelotor has a molecular formula of C₁₉H₁₉N₃O₃ and a molecular weight of 337.4. The chemical structure of voxelotor is:

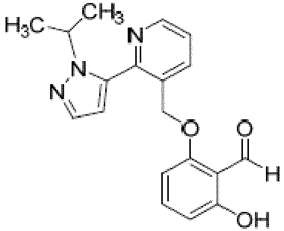

Voxelotor, the active drug substance, is a white-to-yellow-to-beige compound in crystalline Form II of its free base. It is non-hygroscopic. It is highly soluble in common organic solvents such as acetone and toluene and insoluble in water (approximately 0.03 mg/mL).

Each OXBRYTA film-coated tablet for oral use contains 500 mg of voxelotor with the following inactive ingredients: colloidal silicon dioxide, croscarmellose sodium, magnesium stearate, microcrystalline cellulose, and sodium lauryl sulfate. In addition, the film coating contains: polyethylene glycol 3350, polyvinyl alcohol, talc, titanium dioxide, and yellow iron oxide.

### 12 Clinical Pharmacology

### 12.1 Mechanism of Action

Voxelotor is a hemoglobin S (HbS) polymerization inhibitor that binds to HbS with a 1:1 stoichiometry and exhibits preferential partitioning to red blood cells (RBCs). By increasing the affinity of Hb for oxygen, voxelotor demonstrates dose-dependent inhibition of HbS polymerization. Nonclinical studies suggest that voxelotor may inhibit RBC sickling, improve RBC deformability, and reduce whole blood viscosity.

### 12.2 Pharmacodynamics

The pharmacodynamic effect of voxelotor treatment demonstrated a dose-dependent increase in Hb oxygen affinity as determined by the change in p50 (partial pressure of oxygen at which Hb oxygen saturation of 50% is achieved) that was linearly correlated with voxelotor exposure.

The pharmacodynamic effect of voxelotor treatment also demonstrated a dose-dependent reduction in clinical measures of hemolysis (indirect bilirubin and % reticulocytes).

### Cardiac Electrophysiology

At plasma concentrations approximately 2-fold above therapeutic concentrations, voxelotor does not prolong QT interval to any clinically relevant extent.

### 12.3 Pharmacokinetics

Voxelotor is absorbed into plasma and is then distributed predominantly into RBCs due to its preferential binding to Hb. The major route of elimination of voxelotor is by metabolism with subsequent excretion of metabolites into urine and feces. The PK are linear and voxelotor exposures increased proportionally with either single or multiple doses (Table 3) in whole blood, plasma, and RBCs. Steady-state after repeated administration is reached within 8 days and exposures of voxelotor are consistent with accumulation predicted based on single dose data in patients with SCD.

**Table 3: Pharmacokinetics Parameters of Voxelotor in Plasma and Whole Blood**

| **PK Parameter** | **Voxelotor 1,500 mg Geometric Mean (%CV)** |
|---|---|
| **Plasma PK** | |
| AUC₀₋₂₄ₕ (µg·hr/mL) | 246 (27.7) |
| Cₘₐₓ (µg/mL) | 12.6 (24.8) |
| Half-life (hours) | 35.5 (25) |

| **Whole Blood PK** | |
|---|---|
| AUC₀₋₂₄ₕ (µg·hr/mL) | 3820 (35) |
| Cₘₐₓ (µg/mL) | 179 (33.1) |

### Absorption

The median plasma and whole blood Tₘₐₓ of voxelotor after oral administration is 2 hours. The mean peak concentrations in whole blood and RBCs are observed between 6 and 18 hours after oral administration.

### Effect of Food

A high-fat, high-calorie meal increased voxelotor AUC by 42% and Cₘₐₓ by 45% in whole blood relative to AUC and Cₘₐₓ in the fasted state. Similarly, AUC increased by 42% and Cₘₐₓ increased by 95% in plasma.

### Distribution

Voxelotor apparent volume of distribution of the central compartment and peripheral compartment are 338 L and 72.2 L in plasma, respectively. Protein binding is 99.8% in vitro. The blood-to-plasma ratio is approximately 15:1 in patients with SCD.

### Elimination

The geometric mean (%CV) terminal elimination half-life of voxelotor in patients with SCD is 35.5 hours (25%) with concentrations in plasma, whole blood, and RBCs declining in parallel. The apparent oral clearance of voxelotor was estimated as 6.7 L/h in plasma in patients with SCD.

### Metabolism

In vitro and in vivo studies indicate that voxelotor is extensively metabolized through Phase I (oxidation and reduction), Phase II (glucuronidation) and combinations of Phase I and II metabolism. Oxidation of voxelotor is mediated primarily by CYP3A4, with minor contribution from CYP2C19, CYP2B6, and CYP2C9.

### Excretion

Following the administration of radiolabeled voxelotor, approximately 62.6% of the dose and its metabolites are excreted into feces (33.3% unchanged) and 35.5% in urine (0.08% unchanged).

### Specific Populations

No clinically significant differences in the pharmacokinetics of voxelotor were observed based on age (12 to 59 years), sex, body weight (28 to 135 kg), or mild to severe renal impairment (creatinine clearance [CLcr] 15-89 mL/min).

### Pediatric Patients

The pharmacokinetic parameters of voxelotor were similar in pediatric patients 12 to <17 years and adults.

### Patients with Renal Impairment

There was no clinically significant effect of renal function on the excretion of voxelotor. Following a single 900 mg dose of voxelotor, whole blood exposures in subjects with severe renal impairment (eGFR <30 mL/min/1.73 m²) were 25% lower compared to healthy controls.

The unbound plasma concentrations were comparable. OXBRYTA has not been evaluated in patients with end stage renal disease requiring dialysis.

### Patients with Hepatic Impairment

The voxelotor AUC in whole blood were 14% and 15% higher in subjects with mild and moderate hepatic impairment (Child Pugh A and B) and 90% higher in subjects with severe hepatic impairment (Child Pugh C) compared to subjects with normal hepatic function.

### Patients with HbSC Genotype

Voxelotor steady state whole blood AUC and Cmax were 50% and 45% higher in HbSC genotype patients (n=11) compared to HbSS genotype (n=220) patients and voxelotor steady state plasma AUC and Cmax were 23% and 15% higher in HbSC genotype patients compared to HbSS genotype patients.

### Drug Interaction Studies

### Clinical Studies and Model-Informed Approaches

*Effect of Strong CYP3A4 Inhibitors on Voxelotor:* concomitant use of OXBRYTA with ketoconazole is predicted to increase voxelotor AUC in patients by 42% to 83%.

*Effect of Strong or Moderate CYP3A4 Inducers on Voxelotor:* concomitant use of OXBRYTA with rifampin (a strong CYP3A4 inducer) is predicted to decrease voxelotor AUC in patients by up to 77%, and efavirenz (a moderate CYP3A4 inducer) is predicted to decrease voxelotor AUC in patients by up to 60%.

*Effect of Fluconazole on Voxelotor:* concomitant use of OXBRYTA with fluconazole, a moderate CYP3A4 inhibitor, a moderate CYP2C9 inhibitor and a strong CYP2C19 inhibitor, is predicted to increase voxelotor AUC in patients by 40% to 116%.

*Effect of Acid Reducing Agents on Voxelotor:* co-administration of omeprazole (proton pump inhibitor) with OXBRYTA did not alter voxelotor exposure.

*Effect of Voxelotor on CYP450 Enzymes:* in vivo voxelotor inhibits CYP3A4, but not CYP1A2, CYP2C9, CYP2C19, CYP2C8, or CYP2D6. The observed exposure increase of the CYP3A4 substrate midazolam in healthy subjects was 1.6-fold and the predicted increase in patients after multiple dosing is 2-fold.

*Effect of Voxelotor on P-gp:* concomitant use of OXBRYTA with digoxin (a P-gp substrate) did not alter digoxin to a clinically relevant extent.

### In Vitro Studies

*CYP Enzymes:* voxelotor is a reversible and time-dependent inhibitor as well as an inducer of CYP2B6.

*Transporter Systems:* voxelotor is not an inhibitor of P-gp, BCRP, OATP1B1, OATP1B3, OCT2, OAT1, OAT3, MATE1, MATE2-K, or BSEP. Voxelotor is not a substrate of P-gp, BCRP, OATP1A2, OATP1B1, OATP1B3, or BSEP.

### 13 Nonclinical toxicology

### 13.1 Carcinogenesis, Mutagenesis, Impairment of Fertility

Voxelotor was not carcinogenic in a 26-week study in RasH2 transgenic mice at oral doses of 30, 150, or 500 mg/kg/day.

Voxelotor was not genotoxic in the reverse mutation bacterial (Ames) test, rat Comet assay, or rat micronucleus assay.

In a fertility and early embryonic development study, voxelotor was administered orally to rats at 15, 50, and 250 mg/kg/day. Males were dosed 28 days prior to mating through cohabitation and females were dosed 14 days prior to mating through gestation Day 7. Voxelotor had no effect on fertility or reproductive function. Sperm motility was decreased and changes in sperm morphology occurred at 250 mg/kg/day (approximately 5-times the human exposure at 1,500 mg/day).

### 14 Clinical Studies

The efficacy and safety of OXBRYTA in sickle cell disease (SCD) was evaluated in HOPE, a randomized, double-blind, placebo-controlled, multicenter trial [NCT 03036813]. In this study, 274 patients were randomized to daily oral administration of OXBRYTA 1,500 mg (N=90), OXBRYTA 900 mg (N=92), or placebo (N=92). Patients were included if they had from 1 to 10 vasoocclusive crisis (VOC) events within 12 months prior to enrollment and baseline hemoglobin (Hb) ≥5.5 to ≤10.5 g/dL. Eligible patients on stable doses of hydroxyurea for at least 90 days were allowed to continue hydroxyurea therapy throughout the study. Randomization was stratified by patients already receiving hydroxyurea (yes, no), geographic region (North America, Europe, Other), and age (12 to <17 years, 18 to 65 years). The trial excluded patients who received red blood cell (RBC) transfusions within 60 days and erythropoietin within 28 days of enrollment, had renal insufficiency, uncontrolled liver disease, were pregnant, or lactating.

The majority of patients had HbSS or HbS/beta⁰-thalassemia genotype (90%) and were receiving background hydroxyurea therapy (65%). The median age was 24 years (range: 12 to 64 years); 46 (17%) patients were 12 to <17 years of age. Median baseline Hb was 8.5 g/dL (5.9 to 10.8 g/dL). One hundred and fifteen (42%) had 1 VOC event and 159 (58%) had 2 to 10 events within 12 months prior to enrollment.

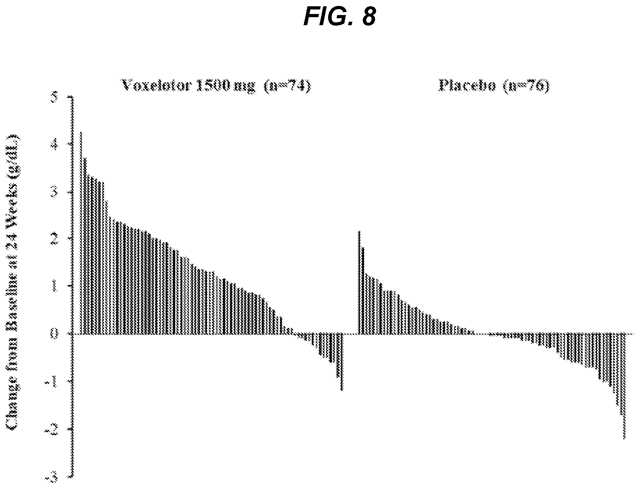

Efficacy was based on Hb response rate defined as a Hb increase of >1 g/dL from baseline to Week 24 in patients treated with OXBRYTA 1,500 mg versus placebo. The response rate for OXBRYTA 1,500 mg was 51.1% (46/90) compared to 6.5% (6/92) in the placebo group (p < 0.001). No outlier subgroups were observed. The distribution of Hb change from baseline for individual patients completing 24 weeks of treatment with OXBRYTA 1,500 mg or placebo is depicted in Figure 8.

Additional efficacy evaluation included change in Hb and percent change in indirect bilirubin and percent reticulocyte count from baseline to Week 24 (Table 4).

**Table 4: Adjusted Mean (SE) Change from Baseline to Week 24 in Hemoglobin and Clinical Measures of Hemolysis**

| | **OXBRYTA 1,500 mg QD (N=90)** | **Placebo (N=92)** | **P Value** |
|---|---|---|---|
| Hemoglobin | 1.14 g/dL (0.13) | -0.08 g/dL (0.13) | < 0.001 |
| Indirect Bilirubin | -29.08 % (3.48) | -3.16 % (3.52) | < 0.001 |
| Percent Reticulocyte Count | -19.93 % (4.60) | 4.54% (4.60) | < 0.001 |

### 16 How Supplied/Storage and Handling

The 500 mg tablet is film-coated, light yellow to yellow, oval shaped, biconvex, debossed with "GBT 500" on one side, and available in:
- Bottles of 90 tablets with child-resistant closure: NDC 72786-101-01

The bottle also contains one desiccant canister and one polyester coil. Do not eat. Store at or below 30°C (86°F).

### 17 Patient Counseling Information

Advise the patient to read the FDA-approved patient labeling (Patient Information).

Advise patients that serious hypersensitivity reactions may occur, and to notify their healthcare providers if they develop generalized rash, urticaria, shortness of breath, facial swelling and eosinophilia *[see Warnings and Precautions (5.1)].*

Advise women not to breastfeed while they are on OXBRYTA therapy *[see Use in Specific Populations (8.2)].*

### Dosage and Administration

Advise patients to:
• Continue taking OXBRYTA every day for as long as their physician tells them. This is a long-term treatment.
• Swallow OXBRYTA tablets whole. Do not cut, crush, or chew the tablets.
• Take with or without food.
• If a dose is missed, continue dosing on the day following the missed dose *[see Dosage and Administration (2.1)].*

**Table 5.**

| **PATIENT INFORMATION OXBRYTA^{™} (ox brye ta) (voxelotor) tablets** | | | |
|---|---|---|---|
| **What is OXBRYTA?** | | | |
| OXBRYTA is a prescription medicine used for the treatment of sickle cell disease in adults and children 12 years of age and older. | | | |
| It is not known if OXBRYTA is safe and effective in children below 12 years of age. | | | |
| **Do not take OXBRYTA** if you have had an allergic reaction to voxelotor or any of the ingredients in OXBRYTA. See the end of this leaflet for a list of the ingredients in OXBRYTA. | | | |
| **If you are receiving exchange transfusions,** talk to your healthcare provider about possible difficulties with the interpretation of certain blood tests when taking OXBRYTA. | | | |
| **Before taking OXBRYTA, tell your healthcare provider about all of your medical conditions, including if you:** | | | |
| | • have liver problems | | |
| | • are pregnant or plan to become pregnant. It is not known if OXBRYTA can harm your unborn baby. | | |
| | • are breastfeeding or plan to breastfeed. It is not known if OXBRYTA can pass into your breastmilk and if it can harm your baby. Do not breastfeed during treatment with OXBRYTA and for at least 2 weeks after the last dose. | | |
| **Tell your healthcare provider about all the medicines you take,** including prescription and over-the-counter medicines, vitamins, and herbal supplements. Some medicines may affect how OXBRYTA works. OXBRYTA may also affect how other medicines work. Keep a list of all your medicines and show it to your healthcare provider. | | | |
| **How should I take OXBRYTA?** | | | |
| | • Take OXBRYTA exactly as your healthcare provider tells you. | | |
| | • Do not change your dose or stop taking OXBRYTA unless your healthcare provider tells you to. | | |
| | • Take OXBRYTA 1 time each day. Swallow each OXBRYTA tablet whole. Do not cut, crush or chew the tablets. | | |
| | | ∘ Your healthcare provider may change your dose if needed. | |
| | • Your healthcare provider may also prescribe hydroxyurea during treatment with OXBRYTA. | | |
| | • Take OXBRYTA with or without food. | | |
| | • If you forget to take a dose of OXBRYTA, skip that dose and return to your normal dosing schedule the next day. | | |
| **What are the possible side effects of OXBRYTA?** | | | |
| **OXBRYTA can cause serious side effects, including:** | | | |
| **Serious allergic reactions.** Tell your healthcare provider or get emergency medical help right away if you get: | | | |
| | | • rash | • shortness of breath |
| | | • hives | • swelling of the face |
| **The most common side effects of OXBRYTA include:** | | | |
| | | • headache | • tiredness |
| | | • diarrhea | • rash |
| | | • stomach (abdominal) pain | • fever |
| | | • nausea | |
| These are not all the possible side effects of OXBRYTA. | | | |
| Call your doctor for medical advice about side effects. | | | |
| **How should I store OXBRYTA?** | | | |
| Store OXBRYTA at or below 86°F (30°C). | | | |
| OXBRYTA comes in a child-resistant package. | | | |
| The bottle contains a desiccant to help keep your medicine dry (protect it from moisture) and polyester coil. Do not eat. | | | |
| **Keep OXBRYTA and all medicines out of the reach of children.** | | | |
| **General information about the safe and effective use of OXBRYTA.** | | | |
| Medicines are sometimes prescribed for purposes other than those listed in a Patient Information leaflet. Do not use OXBRYTA for a condition for which it was not prescribed. Do not give OXBRYTA to other people, even if they have the same symptoms that you have. It may harm them. You can ask your healthcare provider or pharmacist for information about OXBRYTA that is written for health professionals. | | | |
| **What are the ingredients of OXBRYTA?** | | | |
| **Active Ingredient:** voxelotor | | | |
| **Inactive Ingredients:** colloidal silicon dioxide, croscarmellose sodium, magnesium stearate, microcrystalline cellulose, and sodium lauryl sulfate. The film coating contains: polyethylene glycol 3350, polyvinyl alcohol, talc, titanium dioxide, and yellow iron oxide. | | | |
| Manufactured for: Global Blood Therapeutics, Inc. South San Francisco, CA 94080, USA. OXBRYTA is a trademark of Global Blood Therapeutics, Inc. | | | |
| ^{©} 2019 Global Blood Therapeutics, Inc. All rights reserved. | | | |

## Claims

1. Voxelotor for use in a method of treating sickle cell disease in a patient in need thereof, the method comprising administering to the patient 500 mg to 1000 mg of voxelotor per day, wherein the patient has severe hepatic impairment.

2. Voxelotor for use in a method of treating sickle cell disease, as claimed in claim 1, wherein the patient is administered 500 mg of voxelotor per day.

3. Voxelotor for use in a method of treating sickle cell disease, as claimed in claim 1, wherein the patient is administered 1000 mg of voxelotor per day.

4. Voxelotor for use in a method of treating sickle cell disease, as claimed in any one of claims 1-3, wherein voxelotor is administered with food.

5. Voxelotor for use in a method of treating sickle cell disease, as claimed in any one of claims 1-3, wherein voxelotor is administered without food.

6. Voxelotor for use in a method of treating sickle cell disease, as claimed in any one of claims 1-3, wherein voxelotor is administered to the patient after fasting for about 10 hours.

7. Voxelotor for use in a method of treating sickle cell disease, as claimed in any one of claims 1-3, wherein the patient does not eat any food within about 4 hours after voxelotor is administered to the patient.

8. Voxelotor for use in a method of treating sickle cell disease, as claimed in any one of claims 1-7, wherein the patient has a Child-Pugh score of 10-15 points.

9. Voxelotor for use in a method of treating sickle cell disease, as claimed in claim 8, wherein the patient has a Child-Pugh score of 12-15 points.

10. Voxelotor for use in a method of treating sickle cell disease, as claimed in any one of claims 1-9, wherein the patient suffers from a chronic liver disease.

11. Voxelotor for use in a method of treating sickle cell disease, as claimed in any one of claims 1-9, wherein the patient suffers from a condition selected from the group consisting of liver fibrosis, cirrhosis, hepatocellular carcinoma, hepatic inflammation, hepatic steatosis, nonalcoholic fatty liver disease (NAFLD), nonalcoholic steatohepatitis (NASH), portal hypertension, hepatic encephalopathy, hepatitis, and any combination thereof.

12. Voxelotor for use in a method of treating sickle cell disease, as claimed in any one of claims 1-11, wherein voxelotor is administered to the patient once daily.

13. Voxelotor for use in a method of treating sickle cell disease, as claimed in any one of claims 1-12, wherein voxelotor is administered to the patient in an oral formulation.

14. Voxelotor for use in a method of treating sickle cell disease, as claimed in claim 13, wherein the oral formulation is tablet.

15. Voxelotor for use in a method of treating sickle cell disease, as claimed in any one of claims 1-14, wherein voxelotor is administered to the patient in a single oral dose of two tablets daily.

## Patentansprüche

1. Voxelotor zur Verwendung in einem Verfahren zur Behandlung der Sichelzellenkrankheit bei einem Patienten, der eine solche Behandlung benötigt, wobei das Verfahren die Verabreichung von 500 mg bis 1000 mg Voxelotor pro Tag an den Patienten umfasst, wobei der Patient an einer schweren Leberfunktionsstörung leidet.

2. Voxelotor zur Verwendung in einem Verfahren zur Behandlung der Sichelzellenkrankheit, wie in Anspruch 1 beansprucht, wobei dem Patienten 500 mg Voxelotor pro Tag verabreicht werden.

3. Voxelotor zur Verwendung in einem Verfahren zur Behandlung der Sichelzellenkrankheit, wie in Anspruch 1 beansprucht, wobei dem Patienten 1000 mg Voxelotor pro Tag verabreicht werden.

4. Voxelotor zur Verwendung in einem Verfahren zur Behandlung der Sichelzellenkrankheit, wie in einem der Ansprüche1 -3 beansprucht, wobei Voxelotor zusammen mit Nahrung verabreicht wird.

5. Voxelotor zur Verwendung in einem Verfahren zur Behandlung der Sichelzellenkrankheit, wie in einem der Ansprüche1 -3 beansprucht, wobei Voxelotor ohne Nahrung verabreicht wird.

6. Voxelotor zur Verwendung in einem Verfahren zur Behandlung der Sichelzellenkrankheit, wie in einem der Ansprüche1 -3 beansprucht, wobei Voxelotor dem Patienten nach einer Fastenzeit von etwa 10 Stunden verabreicht wird.

7. Voxelotor zur Verwendung in einem Verfahren zur Behandlung der Sichelzellenkrankheit, wie in einem der Ansprüche1 -3 beansprucht, wobei der Patient innerhalb von etwa 4 Stunden nach der Verabreichung von Voxelotor an den Patienten keine Nahrung zu sich nimmt.

8. Voxelotor zur Verwendung in einem Verfahren zur Behandlung der Sichelzellenkrankheit, wie in einem der Ansprüche1 -7 beansprucht, wobei der Patient einen Child-Pugh-Score von 10 bis 15 Punkten aufweist.

9. Voxelotor zur Verwendung in einem Verfahren zur Behandlung der Sichelzellenkrankheit, wie in Anspruch8 beansprucht, wobei der Patient einen Child-Pugh-Score von 12 bis 15 Punkten aufweist.

10. Voxelotor zur Verwendung in einem Verfahren zur Behandlung der Sichelzellenkrankheit, wie in einem der Ansprüche1 -9 beansprucht, wobei der Patient an einer chronischen Lebererkrankung leidet.

11. Voxelotor zur Verwendung in einem Verfahren zur Behandlung der Sichelzellenkrankheit, wie in einem der Ansprüche1 -9 beansprucht, wobei der Patient an einer Erkrankung leidet, die aus der Gruppe ausgewählt ist, bestehend aus Leberfibrose, Leberzirrhose, Leberzellkarzinom, Leberentzündung, Lebersteatose, nichtalkoholischer Fettlebererkrankung (NAFLD), nichtalkoholischer Steatohepatitis (NASH), portaler Hypertonie, hepatischer Enzephalopathie, Hepatitis und einer beliebigen Kombination davon.

12. Voxelotor zur Verwendung in einem Verfahren zur Behandlung der Sichelzellenkrankheit, wie in einem der Ansprüche1 -11 beansprucht, wobei Voxelotor dem Patienten einmal täglich verabreicht wird.

13. Voxelotor zur Verwendung in einem Verfahren zur Behandlung der Sichelzellenkrankheit, wie in einem der Ansprüche1 -12 beansprucht, wobei Voxelotor dem Patienten in einer oralen Formulierung verabreicht wird.

14. Voxelotor zur Verwendung in einem Verfahren zur Behandlung der Sichelzellenkrankheit, wie in Anspruch 13 beansprucht, wobei die orale Formulierung eine Tablette ist.

15. Voxelotor zur Verwendung in einem Verfahren zur Behandlung der Sichelzellenkrankheit, wie in einem der Ansprüche1 -14 beansprucht, wobei Voxelotor dem Patienten in einer einzigen oralen Dosis von zwei Tabletten täglich verabreicht wird.

## Revendications

1. Voxelotor pour une utilisation dans un procédé de traitement de la drépanocytose chez un patient en ayant besoin, le procédé comprenant l'administration au patient de 500 mg à 1000 mg de voxelotor par jour, dans lequel le patient présente une insuffisance hépatique grave.

2. Voxelotor pour une utilisation dans un procédé de traitement de la drépanocytose, selon la revendication 1, dans lequel le patient reçoit 500 mg de voxelotor par jour.

3. Voxelotor pour une utilisation dans un procédé de traitement de la drépanocytose, selon la revendication 1, dans lequel le patient reçoit 1000 mg de voxelotor par jour.

4. Voxelotor pour une utilisation dans un procédé de traitement de la drépanocytose, selon l'une des revendications 1 à 3, dans lequel le voxelotor est administré avec de la nourriture.

5. Voxelotor pour une utilisation dans un procédé de traitement de la drépanocytose, selon l'une des revendications 1 à 3, dans lequel le voxelotor est administré sans nourriture.

6. Voxelotor pour une utilisation dans un procédé de traitement de la drépanocytose, selon l'une des revendications 1 à 3, dans lequel le voxelotor est administré au patient après un jeûne d'environ 10 heures.

7. Voxelotor pour une utilisation dans un procédé de traitement de la drépanocytose, selon l'une des revendications 1 à 3, dans lequel le patient ne consomme pas de nourriture dans les 4 heures environ suivant l'administration de voxelotor au patient.

8. Voxelotor pour une utilisation dans un procédé de traitement de la drépanocytose, selon l'une des revendications 1 à 7, dans lequel le patient présente un score de Child-Pugh de 10 à 15 points.

9. Voxelotor pour une utilisation dans un procédé de traitement de la drépanocytose, selon la revendication 8, dans lequel le patient présente un score de Child-Pugh de 12 à 15 points.

10. Voxelotor pour une utilisation dans un procédé de traitement de la drépanocytose, selon l'une des revendications 1 à 9, dans lequel le patient souffre d'une maladie hépatique chronique.

11. Voxelotor pour une utilisation dans un procédé de traitement de la drépanocytose, selon l'une des revendications 1 à 9, dans lequel le patient souffre d'une affection sélectionnée dans le groupe consistant en fibrose hépatique, cirrhose, carcinome hépatocellulaire, inflammation hépatique, stéatose hépatique, stéatose hépatique non alcoolique (NAFLD), stéatohépatite non alcoolique (NASH), hypertension portale, encéphalopathie hépatique, hépatite et toute combinaison de ceux-ci.

12. Voxelotor pour une utilisation dans un procédé de traitement de la drépanocytose, selon l'une des revendications 1 à 11, dans lequel le voxelotor est administré au patient une fois par jour.

13. Voxelotor pour une utilisation dans un procédé de traitement de la drépanocytose, selon l'une des revendications 1 à 12, dans lequel le voxelotor est administré au patient sous une formulation orale.

14. Voxelotor pour une utilisation dans un procédé de traitement de la drépanocytose selon la revendication 13, dans lequel la formulation orale est un comprimé.

15. Voxelotor pour une utilisation dans un procédé de traitement de la drépanocytose, selon l'une des revendications 1 à 14, dans lequel le voxelotor est administré au patient en une seule dose orale de deux comprimés par jour.
